# EUROPÄISCHE PATENTSCHRIFT

(11) **EP 2 827 924 B1**
(45) Veröffentlichungstag und Bekanntmachung des Hinweises auf die Patenterteilung: **08.05.2019**
(21) Anmeldenummer: 13715131.2
(22) Anmeldetag: 20.03.2013
(51) Int. Cl.: A61M 5/32, A61M 5/158

(54) **SICHERHEITSNADELVORRICHTUNG, INSBESONDERE ZUR PUNKTION VON IN EINEM MENSCHLICHEN ODER TIERISCHEN KÖRPER SUBKUTAN IMPLANTIERTEN PORT**
SAFETY NEEDLE DEVICE, PARTICULARLY FOR THE PUNCTURE OF A PORT IMPLANTED SUBCUTANEOUSLY IN A HUMAN OR ANIMAL BODY
DISPOSITIF D'AIGUILLE DE SÉCURITÉ, EN PARTICULIER POUR LA PONCTION D'UN PORT IMPLANTÉ SOUS LA PEAU D'UN CORPS HUMAIN OU ANIMAL

(30) Priorität: 23.03.2012 DE 102012102519
(43) Veröffentlichungstag der Anmeldung: 28.01.2015
(73) Patentinhaber: PFM Medical AG, 50996 Köln (DE)
(72) Erfinder: KNOBLOCH, Helmut, 52372 Kreuzau (DE)
(74) Vertreter: Hohendorf, Carsten
(86) Internationale Anmeldenummer: PCT/EP2013/000845
(87) Internationale Veröffentlichungsnummer: WO 2013/139476

(56) Entgegenhaltungen:
- US-A1- 2003 114 797
- US-A1- 2005 049 553
- US-A1- 2005 107 748
- US-A1- 2008 262 434

## Beschreibung

Die Erfindung betrifft eine Sicherheitsnadelvorrichtung, insbesondere zur Punktion von in einem menschlichen oder tierischen Körper subkutan implantierten Port, umfassend ein erstes Gehäuse; eine hohle Nadel, wobei die Nadel einen ersten Teilabschnitt und einen zweiten Teilabschnitt aufweist und sich der erste Teilabschnitt entlang einer Punktionsachse erstreckt, die rechtwinklig zu der Oberfläche des menschlichen oder tierischen Körpers verläuft, und der zweite Teilabschnitt der Nadel rechtwinklig zu dem ersten Teilabschnitt angeordnet ist, im Wesentlichen parallel zu der Oberfläche des menschlichen oder tierischen Körpers, wobei die hohle Nadel mit dem ersten Gehäuse verbunden oder verbindbar ist, insbesondere im Bereich des zweiten Teilabschnitts; eine Abdeckung mit einer Durchbrechung zur Aufnahme des ersten Teilabschnitts der Nadel, wobei die Abdeckung von einer ersten Position in eine zweite Position relativ zu dem ersten Gehäuse bewegbar ist, wobei die Bewegung im Wesentlichen entlang der Punktionsachse erfolgt; und ein wenigstens teilweise flexibles Begrenzungselement, zum Begrenzen der Relativbewegung zwischen dem ersten Gehäuse und der Abdeckung in der zweiten Position.

Derartige Sicherheitsnadelvorrichtungen werden zur Verabreichung von Medikamenten eingesetzt, beispielsweise in der Onkologie. Im Rahmen einer Therapie müssen Medikamente häufig und auch über einen längeren Zeitraum verabreicht werden. Um dies möglichst schmerzarm und mit einer minimalen Gewebeschädigung zu erreichen, wird beispielsweise ein Port mit einem Port-Katheter subkutan auf der Brust eines Patienten befestigt. Ein Schlauch des Port-Katheters verbindet den Port mit einer herznahen Vene, um das Medikament schnell in den Blutkreislauf bringen zu können. Der Port-Katheter weist dabei eine Kammer auf, welche mit einer Silikonmembran verschlossen ist. Die Sicherheitsnadelvorrichtung wird durch das Gewebe des menschlichen oder tierischen Körpers und die Membran gestochen, bis die Nadelspitze der Sicherheitsnadelvorrichtung den Boden des Ports erreicht. Die Öffnung der Nadelspitze befindet sich nun in der hohlen Kammer des Ports und das Medikament kann in den Körper des Patienten geleitet werden. Um eine meist schmerzhafte Bewegung der Nadel zu verhindern, wird das Nadelgehäuse mittels Pflaster auf der Haut befestigt. Beim Herausziehen der Nadel muss der Port niedergehalten werden, da er sonst durch die Reibung zwischen Nadel und Membran angehoben wird, was jedoch durch die Verbindung mit dem Gewebe des menschlichen oder tierischen Körpers Schmerzen verursacht. Nach dem Entfernen der Sicherheitsnadelvorrichtung ist die Verletzungsgefahr sehr hoch. Um Nadelstichverletzungen und die damit verbundenen Infektionsgefahren zu verringern, sollte wenigstens die scharfe Nadelspitze kontaktsicher abgedeckt werden. Das Aufsetzen der beim Transport verwendeten Abdeckung von Hand ist nicht dazu geeignet das medizinische Personal, welches die Nadel aus dem Port entfernt vor Verletzungen zu schützen, insbesondere da beim Aufsetzen der beim Transport verwendeten Abdeckung von Hand ein Verletzungsrisiko besteht.

Die EP 1 562 659 B1 offenbart eine Sicherheitsnadelvorrichtung, insbesondere zur Punktion von in einem menschlichen oder tierischen Körper subkutan implantierten Ports. Die Vorrichtung umfasst eine Grundplatte, eine Nadelträgerplatte und eine Abdeckplatte, wobei die Grundplatte und die Nadelträgerplatte jeweils gelenkig mit der Abdeckplatte verbunden sind. An der Nadelträgerplatte ist eine abgewinkelte Nadel vorgesehen, mit einem distalen, perforierenden Schenkel und einem proximalen Schenkel zum Einspeisen eines Medikaments. Die Grundplatte weist eine Durchbrechung auf, durch welche der proximale Schenkel der Nadel durchführbar ist. Weiterhin weist die Abdeckplatte ebenfalls eine Durchbrechung für den distalen Schenkel der Nadel auf. In einem ersten Zustand ist der distale Schenkel der Nadel durch die Durchbrechung in der Grundplatte und durch die Durchbrechung in der Abdeckplatte geführt. In diesem Zustand bilden die Grundplatte und die Nadelträgerplatte einen oberen Teil der Vorrichtung und die Abdeckplatte einen unteren Teil der Vorrichtung, wobei der obere Teil und der untere Teil direkt aufeinander liegen. Der distale Schenkel der Nadel ragt in diesem Zustand nach unten aus der Vorrichtung heraus und kann in den subkutan implantierten Port eingeführt werden. Dazu weist der obere Teil der Vorrichtung zwei Flügel auf, die in eine senkrechte Position klappbar sind, um die Vorrichtung besser in den Port einzuführen. Während der Verwendung der offenbarten Sicherheitsnadelvorrichtung wird die Vorrichtung auf der Oberfläche des menschlichen oder tierischen Körpers mittels Pflaster fixiert. Zum Entfernen der Sicherheitsnadelvorrichtung weist die untere Abdeckplatte ebenfalls zwei Flügel auf, mittels welcher die Abdeckplatte während des Entfernens der Sicherheitsnadelvorrichtung gleichzeitig mit dem Port relativ zu dem menschlichen oder tierischen Körper fixiert werden kann. Zum Herausziehen des distalen Schenkels der Nadel aus dem Port werden die Flügel an dem oberen Teil der Vorrichtung senkrecht aufgestellt und das Bedienpersonal kann mit der anderen Hand den oberen Teil nach oben ziehen, während der untere Teil relativ zu dem menschlichen oder tierischen Körper fixiert wird. Sobald der distale Schenkel der Nadel aus dem menschlichen oder tierischen Körper herausgezogen wurde und die Nadelspitze die Durchbrechung in der Abdeckplatte verlässt, verspringt der distale Schenkel der Nadel durch eine Vorspannung auf ein an der Abdeckplatte vorgesehenes Nadelkissen, so dass die Nadelspitze vor Berührung geschützt ist. Aufgrund der Vorspannung kann es zu einer ungewollten seitlichen Bewegung der Nadelspitze kommen, bevor der distale Schenkel der Nadel den Port-Katheter bzw. den menschlichen oder tierischen Körper verlassen hat.

Aus der WO 03/074112 A1 ist eine Huber-Nadelvorrichtung mit einem Sicherheitsmechanismus bekannt. Die Vorrichtung umfasst ein Gehäuse mit einer gebogenen Durchbrechung für eine Zuführleitung. An dem Ende der gebogenen Durchbrechung ist die Nadel zur Punktion eines in einem menschlichen oder tierischen Körper subkutan implantierten Ports angeordnet. Weiterhin ist innerhalb der gebogenen Durchbrechung eine Schutzhülle angeordnet, welche sich nach dem Betätigen eines Hebels über die Nadel schiebt, um diese vor Berührung zu schützen. Vor dem Entfernen der Vorrichtung aus dem Port wird der Hebel betätigt und während des Herausziehens der Nadel aus dem Port schiebt sich die Schutzhülle automatisch über die Nadel, so dass das Bedienpersonal sich nicht an der Nadelspitze verletzen kann. Nachteilig an dieser Vorrichtung ist die hohe Bauform, welche insbesondere bei Langzeitanwendungen von mehreren Stunden oder Tagen für den Patienten unangenehm ist.

Aus der EP 2 016 964 A1 ist eine Sicherheitsnadelvorrichtung mit einer Abdeckplatte und einem Gehäuse bekannt. Die Vorrichtung umfasst weiterhin eine gebogene Nadel, deren distales Ende durch eine Durchbrechung in der Abdeckplatte in einem ersten Zustand heraustritt und dessen proximales Ende innerhalb des ersten Gehäuses in dem ersten Zustand angeordnet ist. In dem ersten Zustand sind die Abdeckplatte und das Gehäuse der Vorrichtung benachbart zueinander angeordnet. Weiterhin umfasst die Vorrichtung eine Schutzhülle, die im Bereich der Durchbrechung für das distale Ende der Nadel an der Abdeckplatte befestigt ist und in das Gehäuse hineinragt und dabei den gebogenen Nadelschaft umgibt. Beim Herausziehen des distalen Endes der Nadel aus einem Port wird das Gehäuse von der Abdeckplatte senkrecht wegbewegt. Gleichzeitig ist der Nadelschaft die gesamte Zeit von der Hülle umgeben, dessen Ende sich innerhalb des Gehäuses bewegt. Sobald die Nadel komplett aus dem Port herausgezogen wurde, verspringt dessen Spitze, so dass diese nicht ungewollt durch die Durchbrechung in der Abdeckplatte wieder heraustreten kann. Nachteilig an dieser Vorrichtung ist, dass das Herausziehen der Nadel aus dem Port lediglich von der Geschicklichkeit des Bedienpersonals abhängt und dem Patienten insbesondere bei einem schrägen Herausziehen der Nadel vermeidbare Schmerzen zugeführt werden.

Die EP 1 256 355 B1 offenbart einen Sicherheitsschutz, der zur Verwendung mit einer Nadelanordnung angepasst ist, die umfasst: einen Hauptkörper, ein Paar von Schmetterlingsflügeln, die sich von dem Hauptkörper nach außen erstrecken, und eine Nadel, die sich in einem Winkel, vorzugsweise senkrecht, von einem Ende des Hauptkörpers erstreckt und weiterhin umfassend einen länglichen, im Wesentlichen hohlen Schild, wobei der Schild offene Enden und einen länglichen Schlitz aufweist, der sich zwischen den offenen Enden erstreckt; eine mit der Anordnung mit dem Schild verbundene Feder, so dass der Schlitz und die Nadel im Wesentlichen parallel sind, und ein Befestigungsmittel, welches angepasst ist, um den Schild in einer Erstposition im Wesentlichen parallel zu dem Hauptkörper anzuordnen und dem Schild zu ermöglichen, in eine zweite Position durch eine Federkraft verdreht zu werden, um die Nadel zu umgeben und/oder einzufassen.

Die FR 2941867 beschreibt eine Sicherheitsnadelanordnung, insbesondere zur Punktion von einem im menschlichen oder tierischen Körper subkutan implantierten Port umfassend eine Abdeckplatte, eine Nadel und ein Gehäuse. Die Abdeckplatte weist eine Durchbrechung für die Nadel auf, welche an dem Gehäuse befestigt ist. Zwischen dem Gehäuse und der Abdeckplatte ist eine zusammenfaltbare Hülse angeordnet. In einem ersten Zustand, ragt die Nadelspitze durch die Durchbrechung in der Abdeckplatte heraus und kann in dem Port eingeführt werden. In diesem Zustand ist die Hülse zwischen der Abdeckplatte und dem Gehäuse zusammengefaltet angeordnet. Beim Entfernen der Nadel aus dem Port wird die Abdeckplatte relativ zu dem menschlichen oder tierischen Körper fixiert und das Gehäuse wird von der Abdeckplatte entfernt, wodurch sich gleichzeitig die Hülse entfaltet. In einem zweiten Zustand wurde die Nadel vollständig aus dem Port entfernt und ist zwischen der Abdeckplatte und dem Gehäuse innerhalb der auseinandergefalteten Hülse geschützt angeordnet, um ein Wiederaustreten der Nadelspitze aus der Durchbrechung in der Abdeckplatte zu vermeiden, verspringt die Nadelspitze nach dem Herausziehen aus dem Port auf ein an der Abdeckplatte angeordnetes Nadelkissen. Aufgrund der Vorspannung der Nadel zum Verspringen auf das Nadelkissen und der freien Bewegung zwischen der Abdeckplatte und dem Gehäuse während des Herausziehens der Nadel ist es nicht gewährleistet, dass die Nadel senkrecht zu der Oberfläche des menschlichen oder tierischen Körpers aus dem Port herausgezogen wird, was die zuvor genannten Nachteile mitsichbringt.

Aus der US 7,097,637 B2 ist eine Sicherheitsnadelvorrichtung, insbesondere zur Punktion von in einem menschlichen oder tierischen Körper subkutan implantierten Port beschrieben. Die Vorrichtung umfasst eine Abdeckplatte, eine Nadel, ein zweiteiliges zusammenschiebbares Gehäuse und Führungsmittel, welche zwischen Abdeckplatte und Gehäuse angeordnet sind. In einem ersten Zustand ragt die Nadelspitze durch eine Durchbrechung in der Abdeckplatte nach unten aus der Vorrichtung heraus und kann in einen subkutan implantierten Port eingeführt werden. In diesem Zustand weist das Gehäuse eine längliche Form auf. Beim Herausziehen der Nadel aus dem Port wird das zweiteilige Gehäuse mittels der Führungsmittel ineinander geschoben. Vorteilhaft an dieser Ausgestaltung ist, dass die Bewegung der Nadel relativ zu dem Port und dem menschlichen oder tierischen Körper geführt wird. Nachteilig an dieser Ausgestaltung ist jedoch der sehr komplexe Aufbau des Gehäuses und der Führungsmittel zwischen der Abdeckplatte und dem Gehäuse.

Die WO 2007/137339 A1 offenbart eine weitere Variante einer Sicherheitsnadelvorrichtung, insbesondere zur Punktion von in einem menschlichen oder tierischen Körper subkutan implantierten Port. Die Vorrichtung umfasst eine Abdeckplatte mit einer Durchbrechung für eine Nadel, welche in den Port einführbar ist. Die Vorrichtung umfasst weiterhin zwei Flügelelemente, welche jeweils zweiteilig ausgebildet sind und wobei jeweils ein Teil relativ zu dem anderen Teil geführt bewegbar ist. In einem ersten Zustand der Vorrichtung sind die Flügel jeweils auf die Abdeckplatte geklappt und im Wesentlichen parallel zu er Oberfläche des menschlichen oder tierischen Körpers angeordnet und die Nadel tritt durch die Durchbrechung in der Abdeckplatte nach unten heraus und ist in einen Port einführbar. Zum Herausziehen der Nadel aus dem Port werden die Flügel zunächst senkrecht nach oben geklappt und benachbart zueinander angeordnet. Nachfolgend werden die relativ zueinander beweglichen Teile der Flügel senkrecht von der Oberfläche des menschlichen oder tierischen Körpers entfernt, wodurch die Nadel aus dem Port herausgezogen wird. Nach dem Herausziehen der Nadel ist diese in einem Hohlraum zwischen den beiden Flügeln angeordnet und vor einem Kontakt geschützt. Nachteilig an dieser Vorrichtung ist der komplexe Aufbau, insbesondere der zweiteiligen Flügel und die Führung der Flügelteile zueinander.

Die EP 1 011 759 B1 offenbart eine Sicherheitsvorrichtung zur Umhüllung einer medizinischen Nadel, welche umfasst: eine Kanüle mit einer Hohlbohrung, die fest an einer Nadel befestigt ist und zur Herstellung einer medizinischen Nadel zumindest eine scharfe Spitze umfasst; ein geformtes Teil, das gelenkartig mit der Nadel verbunden ist, wobei das geformte Teil eine längliche gestreckte Hülle aufweist, die mehrere in einer Reihe miteinander verbundene, im Wesentlichen steife Segmente umfasst, von welchen jedes mit zumindest einem benachbarten Segment mittels eines Gelenks verbunden ist, wobei jedes der Hülsenelemente eine offene Öffnung, durch welche die Kanüle hindurchgeht, und einen Kanal umfasst, in dem die Kanüle angeordnet ist, wenn die Hülle linear ausgestreckt ist, wobei die Hülle und die Gelenke so angeordnet sind, dass ein Falten der Hülle um die Nadel in einem ersten Zustand, so dass ein Verwendungszugriff auf die scharfe Spitze bei einer medizinischen Prozedur zugelassen ist, um ein Austrecken der Hülle in eine im Wesentlichen ebene Anordnung längst der Kanüle, in der die Kanüle längst des Kanals angeordnet ist, möglich ist, wobei das Ausstrecken der Kanüle ein Drehen jedes eine darin ausgebildete Öffnung für die Kanüle umfassenden Höhlensegments um eine Achse umfasst, welche die Öffnung enthält, wobei die Hülle zumindest ein Arretierelement umfasst, welches mit der Kanüle in Eingriff steht und fest an der Hülle befestigt ist, wobei die Hülle und die Kanüle in der Kombination einen im Wesentlichen steifen Körper bilden, der die scharfe Spitze schützend umschließt und einen Zugriff darauf verhindert. Nachteilig an der Vorrichtung ist zum Einen der komplexe Aufbau der Schutzvorrichtung und zum Anderen die hohe Bauweise der Vorrichtung in der ersten Position. Weiterhin entfaltet sich die Schutzvorrichtung nicht selbstständig, sondern muss nach dem Entfernen der Nadel aus dem menschlichen oder tierischen Körper von dem Bedienpersonal betätigt werden.

Die WO 02/096493 A1 beschreibt eine Sicherheitsnadelvorrichtung mit einem ersten Gehäuse, einem zweiten Gehäuse, einer Nadel und einer zwischen dem ersten und dem zweiten Gehäuse faltbar angeordneten Schutzvorrichtung für den Schaft einer Nadel. Beim Herausziehen der Nadel aus dem menschlichen oder tierischen Körper wird das erste Gehäuse von dem zweiten Gehäuse entfernt, wodurch sich das faltbare Schutzelement entfaltet und um den Schaft der Nadel legt. Nachdem die Nadel aus dem menschlichen oder tierischen Körper entfernt wurde, ist die Spitze innerhalb des zweiten Gehäuses angeordnet und vor einem Kontakt geschützt. Nachteilig an dieser Vorrichtung ist insbesondere die große Bauhöhe, wodurch die Vorrichtung nicht für Langzeitanwendungen geeignet ist, da die Vorrichtung für den Patienten störend wäre.

Die EP 1 430 921 A1 offenbart eine Sicherheitsnadelvorrichtung, insbesondere zur Punktion von in einem menschlichen oder tierischen Körper subkutan implantierten Port umfassend ein erstes Gehäuse und ein zweites Gehäuse, eine Nadel und eine flexible Schutzhülle angeordnet zwischen dem ersten und dem zweiten Gehäuse. In einem ersten Zustand sind das erste Gehäuse und das zweite benachbart zueinander angeordnet und relativ zueinander fixiert. In diesem Zustand ragt die Nadelspitze aus dem zweiten Gehäuse heraus und kann in einen Port eingeführt werden. Die Schutzhülle ist innerhalb eines Hohlraums in dem ersten Gehäuse angeordnet. Zum Herausziehen der Nadel aus dem Port wird diese Verriegelung zwischen dem ersten Gehäuse und dem zweiten Gehäuse gelöst und das zweite Gehäuse wird vom Benutzer relativ zu dem menschlichen oder tierischen Körper fixiert, beispielsweise durch Festhalten. Nachfolgend wird die Nadel aus dem menschlichen oder tierischen Körper herausgezogen, wodurch sich die zwischen dem ersten Gehäuse und dem zweiten Gehäuse angeordnete Schutzhülle entfaltet. In einem zweiten Zustand ist die Nadel vollständig aus dem menschlichen oder tierischen Körper herausgezogen, wobei die Nadelspitze innerhalb des zweiten Gehäuses angeordnet ist und der Nadelschaft durch die Schutzhülle umgeben wird. Nachteilig an dieser Vorrichtung ist insbesondere die Bauhöhe, sodass die Vorrichtung nicht für Langzeitanwendungen am Patienten geeignet ist.

Aus der US 2008/0262434 A1 ist eine weitere Sicherheitsnadelvorrichtung, insbesondere zur Punktion von in einem menschlichen oder tierischen Körper subkutan implantierten Port bekannt. Die Sicherheitsnadelvorrichtung umfasst ein oberes Gehäuse, an welches eine Zuführleitung anschließbar ist und in welchem eine hohle Nadel in einer entsprechenden Halterung angeordnet ist. Die Halterung für die Nadel und die hohle Nadel sind dabei rechtwinkelig zu der Oberfläche des menschlichen oder tierischen Körpers ausgerichtet. Die Vorrichtung umfasst weiter ein unteres Abdeckelement mit einer Durchbrechung für die hohle Nadel und einer Verschlusseinrichtung für die Durchbrechung zum Verschließen der Durchbrechung nachdem die hohle Nadel aus dem menschlichen oder tierischen Körper und der Durchbrechung in der Abdeckplatte gezogen wurde. Zwischen dem oberen Gehäuse und der unteren Abdeckplatte ist eine konische Schutzhülle angeordnet, welche die Nadel nach dem Herausziehen aus dem menschlichen oder tierischen Körper umhüllt. Die konische Schutzhülle besteht aus einer widerstandsfähigen Kunststofffolie. Beim Herausziehen der hohlen Nadel aus dem menschlichen oder tierischen Körper wird die Abdeckplatte relativ zu dem menschlichen oder tierischen Körper fixiert, beispielsweise durch Festhalten. Nachfolgend wird das obere Gehäuse relativ zu der unteren Abdeckplatte bewegt und somit die hohle Nadel aus dem menschlichen oder tierischen Körper gezogen. Zum vollständigen Herausziehen der hohlen Nadel aus dem menschlichen oder tierischen Körper muss die konische Schutzhülle überdehnt werden. Dies hat den Vorteil, dass nach einem Verschließen der Durchbrechung in der unteren Abdeckplatte durch die Verschlusseinrichtung die hohle Nadel durch die überdehnte Schutzhülle gegen die untere Abdeckplatte gedrückt und fixiert wird. Nachteilig an der Vorrichtung ist, dass zum Herausziehen der hohlen Nadel aus dem menschlichen oder tierischen Körper die Schutzhülle überdehnt werden muss, so dass das Bedienpersonal größere Kräfte aufbringen muss. Weiterhin ist während des Herausziehens der Nadel aus dem menschlichen oder tierischen Körper nicht sichergestellt, dass die Nadel senkrecht zu der Oberfläche des menschlichen oder tierischen Körpers herausgezogen wird, wobei ein schräges Herausziehen der Nadel aus dem menschlichen oder tierischen Körper für den Patienten schmerzhaft sein kann. Des Weiteren besteht durch die höheren erforderlichen Kräfte zum Herausziehen der hohlen Nadel aus dem menschlichen oder tierischen Körper die Gefahr, dass das Bedienpersonal das obere Gehäuse und die damit verbundene Nadel aus der rechtwinklig zur Oberfläche des menschlichen oder tierischen Körper verlaufenden Punktionsachse verreißt und die Schutzhülle zwischen oberen Gehäuse und unterer Abdeckplatte beschädigt.

Die US 2005 0107748 A1 offenbart die Merkmale des Oberbegriffs des Anspruchs 1.

Der vorliegenden Erfindung liegt somit die Aufgabe zugrunde eine Sicherheitsnadelvorrichtung, insbesondere zur Punktion von in einem menschlichen oder tierischen Körper subkutan implantierten Port bereitzustellen, welche die Belastungen für den Patienten während des Einführens der Sicherheitsnadelvorrichtung in den Port während der Tragezeit der Sicherheitsnadelvorrichtung und während des Entfernens der Sicherheitsnadelvorrichtung aus dem Port minimiert.

Die Aufgabe wird gelöst durch eine Sicherheitsnadelvorrichtung, insbesondere zur Punktion von in einem menschlichen oder tierischen Körper subkutan implantierten Port, umfassend ein erstes Gehäuse; eine hohle Nadel, wobei die Nadel einen ersten Teilabschnitt und einen zweiten Teilabschnitt aufweist und sich der erste Teilabschnitt entlang einer Punktionsachse erstreckt, die rechtwinklig zu der Oberfläche des menschlichen oder tierischen Körpers verläuft, und der zweite Teilabschnitt der Nadel rechtwinklig zu dem ersten Teilabschnitt angeordnet ist, im Wesentlichen parallel zu der Oberfläche des menschlichen oder tierischen Körpers, wobei die hohle Nadel mit dem ersten Gehäuse verbunden oder verbindbar ist, insbesondere im Bereich des zweiten Teilabschnitts; eine Abdeckung mit einer Durchbrechung zur Aufnahme des ersten Teilabschnitts der Nadel, wobei die Abdeckung von einer ersten Position in eine zweite Position relativ zu dem ersten Gehäuse bewegbar ist, wobei die Bewegung im Wesentlichen entlang der Punktionsachse erfolgt; und ein wenigstens teilweise flexibles Begrenzungselement, zum Begrenzen der Relativbewegung zwischen dem ersten Gehäuse und der Abdeckung in der zweiten Position; welche sich dadurch auszeichnet, dass die Abdeckung auf der dem menschlichen oder tierischen Körper abgewandten Oberfläche eine Führungshülse für den ersten Teilabschnitt der Nadel aufweist, zur Führung der Relativbewegung zwischen der Abdeckung und dem ersten Gehäuse, so dass die Nadel in Richtung der Punktionsachse aus dem Port entfernt wird, wobei die Nadelspitze in der zweiten Position innerhalb der Führungshülse angeordnet ist, und dadurch gekennzeichnet, dass das erste Gehäuse zwei Flügel umfasst, welche aus einer ersten Ausrichtung, welche im Wesentlichen rechtwinklig zur Punktionsachse verläuft, in eine zweite Ausrichtung, im Wesentlichen parallel zur Punktionsachse, klappbar sind, wobei die in der ersten Ausrichtung dem menschlichen oder tierischen Körper abgewandten Oberflächen der Flügel in der zweiten Ausrichtung direkt benachbart zueinander angeordnet und komplementär zueinander ausgebildet sind.

Zum Einführen der erfindungsgemäßen Sicherheitsnadelvorrichtung in einen subkutan im menschlichen oder tierischen Körper implantierten Port sind das erste Gehäuse und die Abdeckung benachbart zueinander angeordnet und gemäß einer besonders zweckmäßigen Variante der Erfindung lösbar miteinander verbunden. In dieser ersten Position tritt der erste Teilabschnitt der Nadel durch die Durchbrechung in der Abdeckung hindurch und verläuft in Richtung der Punktionsachse. In dieser ersten Position kann die Sicherheitsnadelvorrichtung in dem subkutan implantierten Port eingeführt werden bis die Nadelspitze den Boden des Port berührt. In diesem implantierten Zustand kann die erfindungsgemäße Sicherheitsnadelvorrichtung relativ zu dem menschlichen oder tierischen Körper fixiert werden, beispielsweise mittels Klebestreifen oder Pflaster. In diesem implantierten Zustand ist insbesondere die geringe Bauhöhe der erfindungsgemäßen Sicherheitsnadelvorrichtung von Vorteil, da dadurch der Tragekomfort für den Patienten verbessert wird. Nach einer zweckmäßigen Variante der Erfindung weist die Sicherheitsnadelvorrichtung eine maximale Höhe von 15 mm, vorzugsweise eine maximale Höhe von 10 mm auf.

Zum Entfernen der erfindungsgemäßen Sicherheitsnadelvorrichtung aus dem subkutan implantierten Port fixiert das medizinische Personal die Abdeckung beispielsweise mittels einer Hand relativ zu dem menschlichen oder tierischen Körper und bewegt mit der anderen Hand das erste Gehäuse relativ zu der Abdeckung, bis die Abdeckung eine zweite Position relativ zu dem ersten Gehäuse einnimmt. In dieser zweiten Position begrenzt das flexible Begrenzungselement, welches zwischen dem ersten Gehäuse und der Abdeckung angeordnet ist, die Relativbewegung zwischen dem ersten Gehäuse und der Abdeckung. In dieser zweiten Position ist die Nadel der Sicherheitsnadelvorrichtung aus dem subkutan implantierten Port und dem menschlichen oder tierischen Körper entfernt, wobei die Nadelspitze vorzugsweise innerhalb der Führungshülse angeordnet ist. Durch die Führungshülse wird die Relativbewegung zwischen der Abdeckung und dem ersten Gehäuse geführt, so dass die Nadel in Richtung der Punktionsachse aus dem Port entfernt wird. Weiterhin ist in der zweiten Position die Nadelspitze innerhalb der Führungshülse angeordnet, so dass die Nadelspitze vor einem Kontakt isoliert ist und sich das medizinische Personal nicht an der Nadelspitze verletzen kann.

In der ersten Position ist das flexible Begrenzungselement in einem gefalteten Zustand zwischen dem ersten Gehäuse und der Abdeckung angeordnet und entfaltet sich während der Relativbewegung zwischen erstem Gehäuse und Abdeckung, bis es in der zweiten Position eine maximale Ausdehnung erreicht und die Relativbewegung zwischen erstem Gehäuse und Abdeckung begrenzt.

Zweckmäßigerweise ist die Spitze der Nadel mit einem Huberschliff versehen.

In einer zweckmäßigen Variante der erfindungsgemäßen Sicherheitsnadelvorrichtung sind das erste Gehäuse und die Abdeckung in der ersten Position direkt benachbart zueinander angeordnet, wobei die Spitze der Nadel in der ersten Position aus der dem menschlichen oder tierischen Körper zugewandten Oberfläche der Abdeckung austritt. In dieser ersten Position kann die erfindungsgemäße Sicherheitsnadelvorrichtung auf einfache Art und Weise in den subkutan im menschlichen oder tierischen Körper implantierten Port eingeführt werden. Nach einer besonders zweckmäßigen Variante der erfindungsgemäßen Sicherheitsnadelvorrichtung sind das erste Gehäuse und die Abdeckung in der ersten Position und/oder der zweiten Position relativ zueinander fixierbar, so dass es während des Einführens der erfindungsgemäßen Sicherheitsnadelvorrichtung in dem Port oder nach dem Entfernen der Sicherheitsnadelvorrichtung aus dem Port nicht zu einer Relativbewegung zwischen dem ersten Gehäuse und der Abdeckung kommt.

In einer weiteren Variante umfasst die erfindungsgemäße Sicherheitsnadelvorrichtung weiterhin eine Verschlusseinrichtung für die Durchbrechung in der Abdeckung, welche die Durchbrechung für die Nadel in der zweiten Position verschließt. Nach dem Entfernen der erfindungsgemäßen Sicherheitsnadelvorrichtung aus dem subkutan im menschlichen oder tierischen Körper implantierten Port befindet sich die Abdeckung der Sicherheitsnadelvorrichtung in der zweiten Position relativ zu dem ersten Gehäuse der Sicherheitsnadelvorrichtung und die Spitze der hohlen Nadel ist innerhalb der Führungshülse angeordnet. Damit die Spitze der hohlen Nadel nicht wieder durch die Durchbrechung in der Abdeckung heraustreten kann, wird die Durchbrechung durch die Verschlusseinrichtung verschlossen. Dadurch wird das Verletzungsrisiko für das medizinische Personal und den Patienten weiter verringert.

In einer zweckmäßigen Variante der erfindungsgemäßen Sicherheitsnadelvorrichtung ist die Verschlusseinrichtung als Feder ausgebildet, welche in der ersten Position zwischen einer Oberfläche der Abdeckung, vorzugsweise einer Wandung, und der Nadel angeordnet ist und wobei sich die Feder bei Erreichen der zweiten Position, in welcher die Nadel nicht aus der Durchbrechung in der Abdeckung austritt, entspannt und die Durchbrechung für die Nadel verschließt. Dadurch ist gewährleistet, dass die Nadel nach Erreichen der zweiten Position nicht wieder aus der Durchbrechung in der Abdeckung austreten kann. Weiterhin hat die als Feder ausgebildete Verschlusseinrichtung den Vorteil, dass in der ersten Position die Feder in einem gespannten Zustand an dem Schaft der hohlen Nadel anliegt und eine Haltekraft erzeugt, wodurch die Abdeckung relativ zu dem ersten Gehäuse gehalten wird.

Eine derartige als Feder ausgebildete Verschlusseinrichtung umfasst beispielsweise zwei Schenkel und einen Wicklungsbereich, wobei sich ein Schenkel gegenüber einer Oberfläche der Abdeckung abstützt und der andere Schenkel in der ersten Position der Sicherheitsnadelvorrichtung an dem ersten Teilabschnitt der Nadel anliegt und in der zweiten Position der Sicherheitsnadelvorrichtung die Durchbrechung in der Abdeckung verschließt. Vorzugsweise besteht die Feder dabei aus Federstahl.

Bei Erreichen der zweiten Position wird der erste Teilabschnitt der Nadel aus der Durchbrechung in der Abdeckung entfernt und die Feder entspannt sich, so dass ein Teil der Feder gegen eine weitere Oberfläche der Abdeckung zum Anliegen kommt und in dieser Position die Durchbrechung in der Abdeckung verschließt. Durch das Entspannen der Feder in der zweiten Position kommt ein Teil der Feder in Anschlag mit einer Oberfläche der Abdeckung, wobei die Entspannungsbewegung der Feder dabei derart begrenzt wird, dass die Durchbrechung in der Abdeckung verschlossen wird. Durch das Anschlagen eines Teil der Feder an eine Oberfläche der Abdeckung entsteht ein akustisches Geräusch, welches dem medizinischen Personal signalisiert, dass die Verschlusseinrichtung die Durchbrechung in der Abdeckung verschlossen hat.

Eine als Feder ausgebildete Verschlusseinrichtung besteht beispielsweise aus Federstahl, und wird aus einem drahtartigen Element mit einem Durchmesser von 0,6 mm gebildet. Die aus dem Federstahl gebildete Feder hat zum Beispiel zwei Schenkel mit jeweils einer Länge von 6 mm und zwischen den beiden Schenkeln 2,42 Windungen. Eine alternative Feder wird beispielsweise aus einem drahtartigen Element mit einem Durchmesser von 0,5 mm gebildet, hat zwei Schenkel mit einer Länge von jeweils 4 mm und zwischen den beiden Schenkeln 0,92 Windungen.

In einer vorteilhaften Variante der erfindungsgemäßen Sicherheitsnadelvorrichtung weist das Abdeckelement eine Fixierung für die Verschlusseinrichtung auf, zur Fixierung der Verschlusseinrichtung am Abdeckelement. Die Fixierung ist beispielsweise als zylindrische Erhebung ausgebildet, welche in die Wicklung der Feder eingreift. Alternativ oder zusätzlich können beispielsweise Hohlräume an dem Abdeckelement vorgesehen werden, in welche Teile der Verschlusseinrichtung, beispielsweise ein Schenkel einer Feder, eingreifen.

In einer zweckmäßigen Variante der erfindungsgemäßen Sicherheitsnadelvorrichtung ist das flexible Begrenzungselement derart ausgebildet, dass die Spitze der Nadel, welche am ersten Teilabschnitt der Nadel vorgesehen ist, in der zweiten Position innerhalb der Führungshülse angeordnet ist. Insbesondere ist das flexible Begrenzungselement derart ausgebildet, dass die Spitze der Nadel die Führungshülse nicht in Richtung des ersten Gehäuses verlässt, jedoch gleichzeitig sicherstellt, dass in der zweiten Position die Durchbrechung in der Abdeckung beispielsweise durch die eine Verschlusseinrichtung verschlossen werden kann. Bei einer derartigen Ausgestaltung des flexiblen Begrenzungselements ist sichergestellt, dass die Spitze der Nadel in der zweiten Position bei einer normalen Verwendung der erfindungsgemäßen Sicherheitsnadelvorrichtung innerhalb der Führungshülse angeordnet ist und von dieser vor einem Kontakt mit dem medizinischen Personal oder dem Patienten isoliert ist.

Nach einer Variante der erfindungsgemäßen Sicherheitsnadelvorrichtung ist das Begrenzungselement schlauchförmig ausgebildet und der erste Teilabschnitt der Nadel ist wenigstens teilweise innerhalb des schlauchförmigen Begrenzungselements angeordnet. Insbesondere in der zweiten Position ist der Schaft des ersten Teilabschnitts der hohlen Nadel innerhalb des schlauchförmigen Begrenzungselements angeordnet und vor einem Kontakt mit dem medizinischen Personal oder dem Patienten geschützt.

In einer besonders vorteilhaften Variante der erfindungsgemäßen Sicherheitsnadelvorrichtung ist das schlauchförmige Begrenzungselement konisch ausgebildet, so dass es in der ersten Position besonders flach zusammengelegt werden kann, was sich insbesondere positiv auf die maximale Bauhöhe der erfindungsgemäßen Sicherheitsnadelvorrichtung auswirkt.

In einer alternativen Ausführungsform der erfindungsgemäßen Sicherheitsnadelvorrichtung ist das Begrenzungselement aus wenigstens einem fadenförmigen oder bandförmigen Element ausgebildet. Ein fadenförmiges oder bandförmiges Begrenzungselement hat dabei den Vorteil, dass es in der ersten Position auf einem kleineren Raum verstaut werden kann, als ein schlauchförmiges Begrenzungselement. Zwar isoliert ein fadenförmiges oder bandförmiges Element nicht den Schaft des ersten Teilabschnitts der Nadel vor einem Kontakt mit dem medizinischen Personal oder dem Patienten, jedoch geht von diesem Schaft keine Verletzungsgefahr aus.

Zweckmäßigerweise kann das Begrenzungselement aus einem gewebten oder geflochtenen Material bestehen. Insbesondere kann das Begrenzungselement aus einem Textil, einem Kunststoff oder einem Metall bestehen.

Nach einer Variante der erfindungsgemäßen Sicherheitsnadelvorrichtung ist das Begrenzungselement als geschlitztes Band ausgebildet, wobei die Nadel wenigstens in der ersten Position in dem geschlitzten Bereich des bandförmigen Begrenzungselements angeordnet ist. Dabei kann das Begrenzungselement beispielsweise Filmgelenke aufweisen, welche derart angeordnet sind, dass sich das Begrenzungselement in der ersten Position im Wesentlichen parallel zu der Oberfläche des menschlichen oder tierischen Körpers zusammenlegen lässt. Ein derartiges Begrenzungselement weist beispielsweise starre Segmente auf, welche mittels der Filmgelenke gelenkig miteinander verbunden sind, so dass sich das Begrenzungselement in der ersten Position flach zusammenlegen lässt und bei der relativ Bewegung in die zweite Position selbstständig entfaltet. Um das Begrenzungselement möglichst nahe an der Nadel der Sicherheitsnadelvorrichtung anzuordnen kann das Begrenzungselement einen geschlitzten Bereich aufweisen, in welchem die Nadel wenigstens in der ersten Position angeordnet ist.

In einer bevorzugten Variante der erfindungsgemäßen Sicherheitsnadelvorrichtung weist das Abdeckelement einen Hohlraum auf, zur Aufnahme des Begrenzungselements in der ersten Position. In der ersten Position ist das Begrenzungselement somit durch den Hohlraum vor äußeren Beschädigungen geschützt.

Zweckmäßigerweise ist das Begrenzungselement an dem ersten Gehäuse und/oder dem Abdeckelement befestigt, beispielsweise durch eine Klebverbindung oder eine Klemmverbindung.

Gemäß einer vorteilhaften Variante der erfindungsgemäßen Sicherheitsnadelvorrichtung umfasst das Abdeckelement wenigstens einen Flügel, vorzugsweise zwei Flügel. Die Flügel an dem ersten Gehäuse und/oder dem Abdeckelement fungieren als Handhabungshilfe der erfindungsgemäßen Sicherheitsnadelvorrichtung während des Einführens oder des Herausziehens der Sicherheitsnadelvorrichtung in bzw. aus einem subkutan im menschlichen oder tierischen Körper implantierten Ports oder als Fixierungshilfe zum Fixieren der erfindungsgemäßen Sicherheitsnadelvorrichtung relativ zum menschlichen oder tierischen Körper im eingeführten Zustand.

In einer besonders zweckmäßigen Variante weist der wenigstens eine Flügel wenigstens eine Rippe auf, welche ein besonders griffiges Gefühl für den Benutzer der erfindungsgemäßen Sicherheitsnadelvorrichtung erzeugen soll und um ein Abrutschen eines oder mehrerer Finger des Bedienpersonals während des Einführens bzw. Herausziehens der erfindungsgemäßen Sicherheitsnadelvorrichtung aus dem subkutan im menschlichen oder tierischen Körper implantierten Port zu verhindern.

Nach einer vorteilhaften Variante der erfindungsgemäßen Sicherheitsnadelvorrichtung sind an dem ersten Gehäuse und an der Abdeckung jeweils zwei Flügel angeordnet, wobei die Flügel an dem ersten Gehäuse vorzugsweise größer oder kleiner ausgebildet sind, als die Flügel an der Abdeckung. Somit können vorzugsweise die Flügel unmittelbar dem ersten Gehäuse bzw. der Abdeckung zugeordnet werden. Insbesondere sind die Flügel an dem ersten Gehäuse größer ausgebildet als die Flügel an der Abdeckung, da mittels dieser die Kraft auf die erfindungsgemäße Sicherheitsnadelvorrichtung übertragen wird zum Herausziehen der Nadel aus dem Port, während die Flügel der Abdeckung lediglich dazu dienen, die Abdeckung relativ zu dem menschlichen oder tierischen Körper und dem darin subkutan implantierten Port-Katheter zu fixieren.

Erfindungsgemäß sind die Flügel aus einer ersten Ausrichtung, welche im Wesentlichen rechtwinklig zur Punktionsachse verläuft, in eine zweite Ausrichtung, im Wesentlichen parallel zur Punktionsachse, klappbar. Somit lassen sich beispielsweise die Flügel an dem ersten Gehäuse zum Herausziehen der Nadel aus dem subkutan im menschlichen oder tierischen Körper implantierten Port in die zweite Ausrichtung klappen, so dass zum Herausziehen der Nadel eine Kraft entlang der Punktionsachse einfacher aufgebracht werden kann.

Erfindungsgemäß sind die in der ersten Ausrichtung die dem menschlichen oder tierischen Körper abgewandten Oberflächen der Flügel in der zweiten Ausrichtung direkt benachbart zueinander angeordnet, wobei die benachbart zueinander angeordneten Oberflächen der Flügel komplementär zueinander ausgebildet sind. Dies verbessert insbesondere das Bediengefühl während des Herausziehens der Nadel aus dem Port, da die t benachbart zueinander angeordneten Flügel das Gefühl vermitteln, dass es sich um ein einziges Element handele und sich die Flügel nicht relativ zueinander bewegen.

In einer besonders bevorzugten Variante der erfindungsgemäßen Sicherheitsnadelvorrichtung weist das erste Gehäuse wenigstens eine Ausbuchtung, vorzugsweise zwei Ausbuchtungen auf, zur Aufnahme von Fingern des Bedienpersonals während der Punktion des implantierten Port. Zweckmäßigerweise ist die wenigstens eine Ausbuchtung im Bereich der Punktionsachse der erfindungsgemäßen Sicherheitsnadelvorrichtung angeordnet.

Die wenigstens eine Ausbuchtung dient als Handhabungshilfe für das Bedienpersonal während des Einführens der Nadel in den subkutan im menschlichen oder tierischen Körper implantierten Port. Durch die Anordnung der wenigstens einen Ausbuchtung im Bereich der Punktionsachse lässt sich die erfindungsgemäße Sicherheitsnadelvorrichtung genau im Bereich des Ports platzieren.

Die wenigstens eine Ausbuchtung ist beispielsweise unterhalb der zwei klappbaren Flügel des ersten Gehäuses angeordnet, welche während des Einführens bzw. Herausziehens der Nadel aus dem Port parallel zur Punktionsachse geklappt werden und in dieser Position die wenigstens eine Ausbuchtung freigeben.

Nach einer weiteren vorteilhaften Variante der erfindungsgemäßen Sicherheitsnadelvorrichtung umfasst die Abdeckung Seitenwände und einen Deckel, welche zusammen mit der Abdeckung ein zweites Gehäuse ausbilden. Dieses zweite Gehäuse schützt beispielsweise das Begrenzungselement und/oder die Verschlusseinrichtung vor einer äußeren Beschädigung und/oder Verschmutzung. Zweckmäßigerweise weist der Deckel des zweiten Gehäuses der erfindungsgemäßen Sicherheitsnadelvorrichtung eine erste Durchbrechung für die Nadel, insbesondere den ersten Teilabschnitt der Nadel, und eine zweite Durchbrechung für das flexible Begrenzungselement auf.

Gemäß einer weiteren Variante der erfindungsgemäßen Sicherheitsnadelvorrichtung weist die Nadel eine auf dem beispielsweise subkutan im menschlichen oder tierischen Körper implantierten Port angepasste Länge auf, insbesondere ist die Länge des ersten Teilabschnitts der Nadel auf dem Port abgestimmt. Dadurch kann gewährleistet werden, dass die Spitze des ersten Teilabschnitts der Nadel den Boden des Port berührt und die Abdeckung auf der Oberfläche des menschlichen oder tierischen Körpers angeordnet ist, um dort fixiert zu werden. Nach einer zweckmäßigen Variante weisen das erste Gehäuse und/oder die Abdeckung und der Port eine übereinstimmende Farbmarkierung auf. Somit lässt sich auf einfache Art und Weise eine zu dem subkutan im menschlichen oder tierischen Körper implantierten Port passende Sicherheitsnadelvorrichtung auswählen.

Nach einer weiteren vorteilhaften Variante der erfindungsgemäßen Sicherheitsnadelvorrichtung ist die Abdeckung und/oder das erste Gehäuse wenigstens teilweise transparent ausgebildet, insbesondere derart, dass die Punktionsstelle in den menschlichen oder tierischen Körper und den darin subkutan implantierten Port während der Punktion für das Bedienpersonal sichtbar ist. Somit lässt sich die erfindungsgemäße Sicherheitsnadelvorrichtung wesentlich genauer platzieren und in den subkutan implantierten Port einführen.

Zweckmäßigerweise wird eine erfindungsgemäße Sicherheitsnadelvorrichtung mit wenigstens einem Flügel an dem ersten Gehäuse und/oder der Abdeckung mit dem wenigstens einen Flügel in der ersten Position hergestellt, damit der wenigstens eine Flügel im eingeführten Zustand der erfindungsgemäßen Sicherheitsnadelvorrichtung nicht automatisch in Richtung der Punktionsachse klappt. Dadurch lässt sich die erfindungsgemäße Sicherheitsnadelvorrichtung einfacher an der Oberfläche des menschlichen oder tierischen Körpers befestigen.

Nachfolgend wird die Erfindung anhand von in den Figuren dargestellten Ausführungsbeispielen nähe erläutert. Es zeigen:
- Figur 1: eine perspektivische Ansicht einer ersten Ausführungsform einer erfindungsgemäßen Sicherheitsnadelvorrichtung in einer ersten Position,
- Figur 2: eine perspektivische Ansicht der erfindungsgemäßen Sicherheitsnadelvorrichtung aus Figur 1 in einer zweiten Position,
- Figur 3: eine perspektivische Ansicht einer zweiten Ausführungsform einer erfindungsgemäßen Sicherheitsnadelvorrichtung in der ersten Position,
- Figur 4: eine perspektivische Ansicht der erfindungsgemäßen Sicherheitsnadelvorrichtung aus Figur 3 in der zweiten Position,
- Figur 5: eine perspektivische Ansicht einer dritten Ausführungsform einer erfindungsgemäßen Sicherheitsnadelvorrichtung in einer Zwischenposition,
- Figur 6: eine Ansicht von der dem menschlichen oder tierischen Körper zugewandten Seite auf die erfindungsgemäße Sicherheitsnadelvorrichtung aus Figur 5 in der ersten Position,
- Figur 7: eine Ansicht von der dem menschlichen oder tierischen Körper zugewandten Seite auf die erfindungsgemäße Sicherheitsnadelvorrichtung aus Figur 5 in der Zwischenposition,
- Figur 8: eine Ansicht von der dem menschlichen oder tierischen Körper zugewandten Seite auf die erfindungsgemäße Sicherheitsnadelvorrichtung aus Figur 5 in der zweiten Position,
- Figur 9: eine perspektivische Ansicht einer vierten Ausführungsform einer erfindungsgemäßen Sicherheitsnadelvorrichtung in der ersten Position,
- Figur 10: eine perspektivische Ansicht der erfindungsgemäßen Sicherheitsnadelvorrichtung aus Figur 9 in der zweiten Position,
- Figur 11: eine Seitenansicht einer fünften Ausführungsform einer erfindungsgemäßen Sicherheitsnadelvorrichtung in der ersten Position,
- Figur 12: eine Seitenansicht der erfindungsgemäßen Sicherheitsnadelvorrichtung aus Figur 11 in der Zwischenposition,
- Figur 13: eine perspektivische Ansicht der erfindungsgemäßen Sicherheitsnadelvorrichtung aus Figur 11 in der zweiten Position,
- Figur 14: eine perspektivische Ansicht einer sechsten Ausführungsform einer erfindungsgemäßen Sicherheitsnadelvorrichtung in der Zwischenposition,
- Figur 15: eine perspektivische Ansicht der erfindungsgemäßen Sicherheitsnadelvorrichtung aus Figur 14 in der zweiten Position,
- Figur 16: eine seitliche Detailansicht einer Abdeckung von einer erfindungsgenmäßen Sicherheitsnadelvorrichtung,
- Figur 17: eine Detailansicht von der dem menschlichen oder tierischen Körper zugewandten Seite auf die Abdeckung aus Figur 16,
- Figur 18: eine Detailansicht gemäß Figur 17 mit einer in der Abdeckung angeordneten Verschlusseinrichtung,
- Figur 19: Detailansichten einer als Feder ausgebildeten Verschlusseinrichtung zur Verwendung mit einer erfindungsgemäßen Sicherheitsnadelvorrichtung,
- Figur 20: eine Detailansicht eines ersten Gehäuses von einer erfindungsgemäßen Sicherheitsnadelvorrichtung und
- Figur 21: eine Schnittansicht von gefalteten Flügeln eines ersten Gehäuses von einer erfindungsgemäßen Sicherheitsnadelvorrichtung.

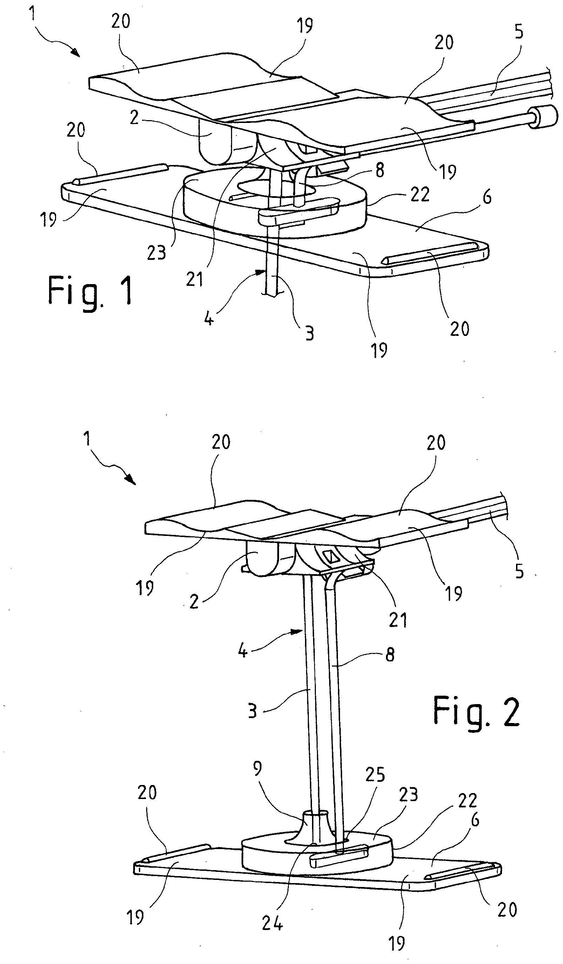

In Figur 1 ist eine perspektivische Ansicht einer ersten Ausführungsform einer erfindungsgemäßen Sicherheitsnadelvorrichtung 1 insbesondere zur Punktion von in einem menschlichen oder tierischen Körper subkutan implantierten Port, in einer ersten Position dargestellt. Die erfindungsgemäße Sicherheitsnadelvorrichtung 1 umfasst ein erstes Gehäuse 2 und eine hohle Nadel 3, wobei die Nadel 3 einen ersten Teilabschnitt 4 und einen zweiten Teilabschnitt 5 aufweist und sich der erste Teilabschnitt 4 entlang einer Punktionsachse erstreckt, die rechtwinklig zu der Oberfläche des menschlichen oder tierischen Körpers verläuft und der zweite Teilabschnitt 5 der Nadel 3 rechtwinklig zu dem ersten Teilabschnitt 4 angeordnet ist, im Wesentlichen parallel zu der Oberfläche des menschlichen oder tierischen Körpers, wobei die hohle Nadel 3 mit dem ersten Gehäuse 2 verbunden oder verbindbar ist, insbesondere im Bereich des zweiten Teilabschnitts 5. Die erfindungsgemäße Sicherheitsnadelvorrichtung 1 gemäß Figur 1 umfasst weiterhin eine Abdeckung 6 mit einer Durchbrechung 7 zur Aufnahme des ersten Teilabschnitts 4 der Nadel 3, wobei die Abdeckung 6 von einer ersten Position in eine zweite Position relativ zu dem ersten Gehäuse 2 bewegbar ist, wobei die Bewegung im Wesentlichen entlang der Punktionsachse erfolgt. Weiterhin umfasst die erfindungsgemäße Sicherheitsnadelvorrichtung 1 aus Figur 1 ein wenigstens teilweise flexibles Begrenzungselement 8, zum Begrenzen der Relativbewegung zwischen dem ersten Gehäuse 2 und der Abdeckung 6 in der zweiten Position. Auf der dem menschlichen oder tierischen Körper abgewandten Oberfläche weist die Abdeckung 6 eine Führungshülse 9 für den ersten Teilabschnitt 4 der Nadel 3 auf.

In der ersten Position sind das erste Gehäuse 2 und die Abdeckung 6 direkt benachbart zueinander angeordnet, wobei die Spitze der Nadel 3 in der ersten Position aus der dem menschlichen oder tierischen Körper zugewandten Oberfläche der Abdeckung 6 austritt. In dieser ersten Position wird die dargestellte erfindungsgemäße Sicherheitsnadelvorrichtung in einen in einem menschlichen oder tierischen Körper subkutan implantierten Port eingeführt.

In dem eingeführten Zustand kann die erfindungsgemäße Sicherheitsnadelvorrichtung 1 relativ zu dem menschlichen oder tierischen Körper fixiert werden, beispielsweise durch Festkleben der erfindungsgemäßen Sicherheitsnadelvorrichtung auf der Oberfläche des menschlichen oder tierischen Körpers beispielsweise mittels Pflaster.

In der in Figur 1 dargestellten ersten Position sind das erste Gehäuse 2 und die Abdeckung 6 relativ zueinander fixiert, wie nachfolgend näher erläutert wird.

Zum Verschließen der Durchbrechung 7 in der zweiten Position umfasst die erfindungsgemäße Sicherheitsnadelvorrichtung 1 weiterhin eine Verschlusseinrichtung 10 für die Durchbrechung 7 in der Abdeckung 6. Die Verschlusseinrichtung 10 ist beispielsweise als Feder 12 ausgebildet, welche in der ersten Position zwischen einer Oberfläche der Abdeckung 6, vorzugsweise einer Wandung 11 und der Nadel 3 angeordnet ist und wobei sich die Feder 12 bei Erreichen der zweiten Position, in welcher die Nadel 3 nicht aus der Durchbrechung 7 in der Abdeckung 6 austritt, entspannt und die Durchbrechung 7 für die Nadel 3 verschließt. In der ersten Position ist die Feder 12 zwischen einer Wandung 11 der Abdeckung 6 und dem Schaft des ersten Teilabschnitts 4 der Nadel 3 gespannt, wodurch eine Reibkraft zwischen dem Schaft der Nadel 3 und der Feder entsteht. Durch diese Reibkraft ist das erste Gehäuse 2 relativ zu der Abdeckung 6 fixiert. Durch Aufbringen einer externen Kraft durch einen Benutzer kann die in Figur 1 dargestellte erfindungsgemäße Sicherheitsnadelvorrichtung 1 von der ersten Position in die in Figur 2 dargestellte zweite Position überführt werden, wodurch die Nadel 3 aus dem Port und dem menschlichen oder tierischen Körper herausgezogen wird.

In der in Figur 2 dargestellten Position hat das Begrenzungselement 8 die Relativbewegung zwischen dem ersten Gehäuse 2 und der Abdeckung 6 begrenzt. In dieser zweiten Position ist die Spitze der Nadel 3 innerhalb der Führungshülse 9 der Abdeckung 6 angeordnet und dort vor einem Kontakt mit dem Benutzer oder dem menschlichen oder tierischen Körper des Patienten isoliert.

Die als Feder 12 ausgebildete Verschlusseinrichtung 10 umfasst zwei Schenkel 13 und einen Wicklungsbereich 14, wobei sich ein Schenkel 13 gegenüber einer Oberfläche der Abdeckung 6 abstützt und der andere Schenkel 13 in der ersten Position der erfindungsgemäßen Sicherheitsnadelvorrichtung 1 an den ersten Teilabschnitt 4 der Nadel 3 anliegt und in der zweiten Position der erfindungsgemäßen Sicherheitsnadelvorrichtung 1 die Durchbrechung 7 in der Abdeckung 6 verschließt. Dadurch wird gewährleistet, dass nach Erreichen der zweiten Position die Spitze der Nadel 3 nicht wieder aus der Durchbrechung 7 in der Abdeckung 6 austreten kann, was die Sicherheit der erfindungsgemäßen Sicherheitsnadelvorrichtung 1 deutlich erhöht.

Das Abdeckelement 6 der erfindungsgemäßen Sicherheitsnadelvorrichtung 1 aus den Figuren 1 und 2 weist weiterhin eine Fixierung 15 für die Verschlusseinrichtung 10 auf. Zur Fixierung der Verschlusseinrichtung 10 am Abdeckelement 6, beispielsweise in Form einer zylindrischen Erhebung, die in die Wicklung 14 der Feder 12 eingreift.

Das flexible Begrenzungselement 8 der erfindungsgemäßen Sicherheitsnadelvorrichtung 1 ist derart ausgebildet, dass die Spitze der Nadel 3 in der zweiten Position innerhalb der Führungshülse 9 angeordnet ist. Insbesondere weist das flexible Begrenzungselement 8 eine geringe Elastizität auf, so dass selbst bei Aufbringen einer größeren Kraft die Spitze der Nadel 3 jederzeit in der Führungshülse 9 der Abdeckung 3 verbleibt. Das Begrenzungselement 8 der erfindungsgemäßen Sicherheitsnadelvorrichtung 1 aus den Figuren 1 und 2 weist einen runden Querschnitt auf und besteht beispielsweise aus Kunststoff. Alternativ kann das Begrenzungselement 8 ebenfalls aus einem fadenförmigen Element gebildet werden, welches zum Beispiel aus einem gewebten oder geflochtenen Material, insbesondere einem Textil, einem Kunststoff oder einem Metall besteht. Das Begrenzungselement 8 ist an der Abdeckung 6 befestigt und an dem ersten Gehäuse 2 in einer Führung fixiert, wobei die Führung und das Begrenzungselement 8 derart ausgebildet sind, dass die Relativbewegung zwischen dem ersten Gehäuse 2 und der Abdeckung 6 in der zweiten Position begrenzt wird.

Das erste Gehäuse 2 und die Abdeckung 6 der erfindungsgemäßen Sicherheitsnadelvorrichtung 1 aus den Figuren 1 und 2 umfassen jeweils zwei Flügel 19 zur besseren Handhabung der erfindungsgemäßen Sicherheitsnadelvorrichtung 1 während des Einführens oder Herausziehens der erfindungsgemäßen Sicherheitsnadelvorrichtung 1 in bzw. aus einem subkutan im menschlichen oder tierischen Körper implantierten Port. Die Flügel 19 des ersten Gehäuses 2 und der Abdeckung 6 weisen jeweils eine Rippe 20 auf, um den Benutzer ein besonders griffiges Gefühl zu vermitteln.

Die Flügel 19 des ersten Gehäuses 2 sind aus einer ersten Ausrichtung, welche im Wesentlichen rechtwinklig zur Punktionsachse verläuft, in eine zweite Ausrichtung, im Wesentlichen parallel zur Punktionsachse klappbar. In der zweiten Ausrichtung sind die in der ersten Ausrichtung dem menschlichen oder tierischen Körper abgewandten Oberflächen der Flügel 19 direkt benachbart zueinander angeordnet, wobei die benachbart zueinander angeordneten Oberflächen der Flügel 19 vorzugsweise komplementär zueinander ausgebildet sind.

Weiterhin weist das erste Gehäuse 2 zwei Ausbuchtungen 21 auf, zur Aufnahme von Fingern des Bedienpersonals während der Punktion des im menschlichen oder tierischen Körper implantierten Ports. Die Ausbuchtungen 21 sind unterhalb der klappbaren Flügel 19 des ersten Gehäuses 2 im Bereich der Punktionsachse angeordnet und sind nach dem Klappen der Flügel 19 in die zweite Ausrichtung für das Bedienpersonal zugängig.

Die Abdeckung 6 umfasst weiterhin Seitenwände 22 und einen Deckel 23, welche zusammen mit der Abdeckung 6 ein zweites Gehäuse ausbilden. Der Deckel 23 des zweiten Gehäuses weist eine erste Durchbrechung 24 für die Nadel 3 und eine zweite Durchbrechung 25 für das flexible Begrenzungselement 8 auf.

Die Nadel 3 der erfindungsgemäßen Sicherheitsnadelvorrichtung 1 aus den Figuren 1 und 2 weist vorzugsweise eine auf den subkutan im menschlichen oder tierischen Körper implantierten Port angepasste Länge auf. Zweckmäßigerweise weisen das erste Gehäuse 2 und/oder die Abdeckung 6 der erfindungsgemäßen Sicherheitsnadelvorrichtung 1 aus den Figuren 1 und 2 und der im menschlichen oder tierischen Körper subkutan implantierte Port eine übereinstimmende Farbmarkierung auf.

Gemäß einer weiteren vorteilhaften Ausgestaltung der erfindungsgemäßen Sicherheitsnadelvorrichtung 1 sind die Abdeckung 6 und/oder das Gehäuse 2 wenigstens teilweise transparent ausgebildet, insbesondere derart, dass während der Punktion des Ports die Einstichstelle für das Bedienpersonal sichtbar ist.

Der zweite Teilabschnitt 5 der Nadel 3 kann innerhalb des ersten Gehäuses 2 enden und dort mit einer Zuführleitung verbunden sein oder rechtwinklig zu der Punktionsachse das erste Gehäuse 2 verlassen und nachfolgend mit einer Zuführleitung verbunden werden.

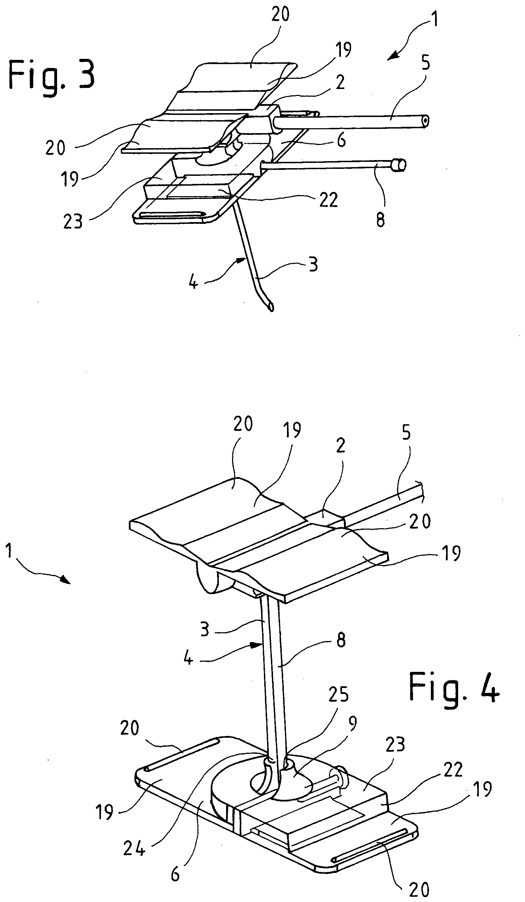

Figur 3 zeigt eine perspektivische Ansicht einer zweiten Ausführungsform einer erfindungsgemäßen Sicherheitsnadelvorrichtung 1 in der ersten Position und Figur 2 zeigt eine perspektivische Ansicht der Sicherheitsnadelvorrichtung 2 aus Figur 3 in der zweiten Position. Die zweite Ausführungsform der erfindungsgemäßen Sicherheitsnadelvorrichtung 1 aus den Figuren 3 und 4 unterscheidet sich von der ersten Ausführungsform der erfindungsgemäßen Sicherheitsnadelvorrichtung 1 aus dem Figuren 1 und 2 dadurch, dass das Begrenzungselement 8 fest mit dem ersten Gehäuse 2 verbunden ist und im Bereich der Abdeckung 6 in einer Führung gelagert ist. Das Begrenzungselement 8 und die Führung sind derart ausgebildet, dass die Relativbewegung zwischen dem ersten Gehäuse 2 und der Abdeckung 6 in der zweiten Position begrenzt wird. Die Führung des Begrenzungselements 8 im Bereich der Abdeckung 6 führt das Begrenzungselement 8 seitlich aus dem zweiten Gehäuse heraus, welches durch die Abdeckung 6, die Seitenwände 22 und den Deckel 23 gebildet wird. Im Übrigen stimmt die zweite Ausführungsform gemäß den Figuren 3 und 4 mit der ersten Ausführungsform gemäß den Figuren 1 und 2 überein.

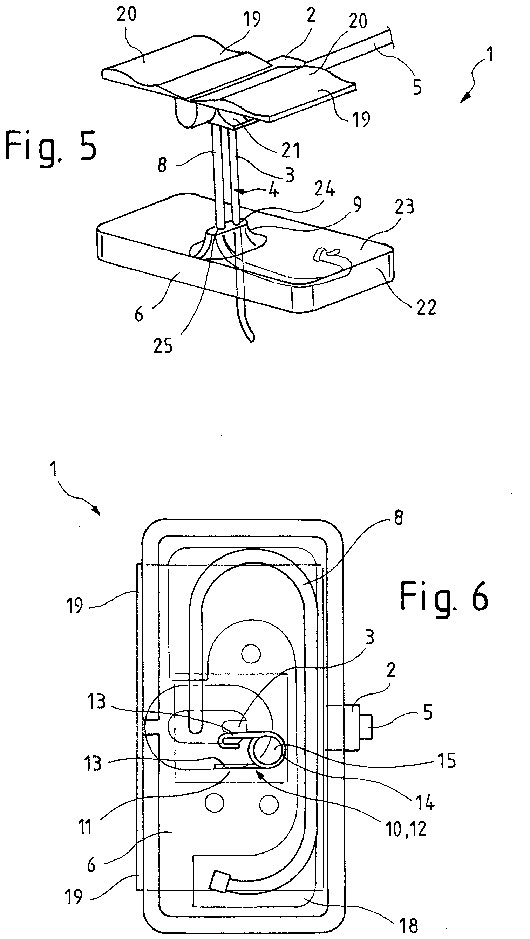

In Figur 5 ist eine perspektivische Ansicht einer dritten Ausführungsform einer erfindungsgemäßen Sicherheitsnadelvorrichtung 1 in einer Zwischenposition dargestellt. Die dritte Ausführungsform gemäß Figur 5 unterscheidet sich von der zweiten Ausführungsform gemäß den Figuren 3 und 4 dadurch, dass das Begrenzungselement 8 innerhalb der als zweites Gehäuse ausgebildeten Abdeckung 6 geführt wird und nicht seitlich nach außen gemäß dem zweiten Ausführungsbeispiel der erfindungsgemäßen Sicherheitsnadelvorrichtung 1 aus den Figuren 3 und 4. Die Abdeckung 6 der erfindungsgemäßen Sicherheitsnadelvorrichtung 1 aus Figur 5 weist einen Hohlraum 18 auf, zur Aufnahme des Begrenzungselements 8 in der ersten Position. Beim Übergang von der ersten Position in die zweite Position wird das Begrenzungselement 8 kontinuierlich aus dem Hohlraum 18 über die zweite Durchbrechung 8 in dem Deckel 23 des zweiten Gehäuses herausgezogen, bis es in der zweiten Position die Relativbewegung zwischen dem ersten Gehäuse 2 und der Abdeckung 6 begrenzt und nicht weiter durch die Durchbrechung 25 im Deckel 23 herausgezogen werden kann.

In Figur 6 ist eine Ansicht von der dem menschlichen oder tierischen Körper zugewandten Seite auf die erfindungsgemäße Sicherheitsnadelvorrichtung 1 aus Figur 5 in der ersten Position dargestellt. In dieser ersten Position ist das Begrenzungselement 8 innerhalb des Hohlraums 18 der Abdeckung 6 angeordnet. Weiterhin lässt sich der Figur 6 die als Verschlusseinrichtung 10 ausgebildete Feder 12 entnehmen. Die Feder 12 weist zwei Schenkel 13 und einen Wicklungsbereich 14 auf. In der dargestellten ersten Position ist die Feder zwischen einer Wandung 11 der Abdeckung 6 und dem Schaft des ersten Teilabschnitts 4 der Nadel 3 gespannt und zusätzlich mittels der Fixierung 15 an der Abdeckung 6 fixiert. Die Fixierung 15 ist als zylindrischer Vorsprung ausgebildet, welcher in die Wicklung 14 der Feder 12 eingreift.

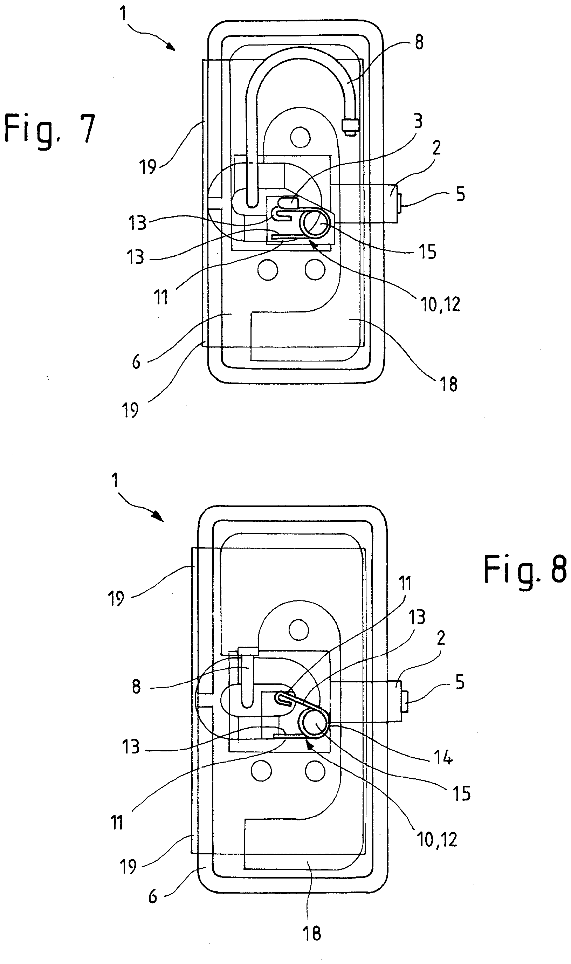

In Figur 7 ist eine Ansicht von der dem menschlichen oder tierischen Körper zugewandten Seite auf die erfindungsgemäße Sicherheitsnadelvorrichtung 1 aus Figur 5 in der Zwischenposition dargestellt. In dieser Zwischenposition wurde das Begrenzungselement 8 teilweise aus der Abdeckung 6 herausgezogen, da das erste Gehäuse 2 sich von der Abdeckung 6 entfernt hat. Gleichzeitig wurde die Nadel 3 teilweise aus dem subkutan im menschlichen oder tierischen Körper implantierten Port herausgezogen.

In Figur 8 ist eine Ansicht von der dem menschlichen oder tierischen Körper zugewandten Seite auf die erfindungsgemäße Sicherheitsnadelvorrichtung 1 aus Figur 5 in der zweiten Position dargestellt. In der zweiten Position hat das Begrenzungselement 8 die Relativbewegung zwischen dem ersten Gehäuse 2 und der Abdeckung 6 der erfindungsgemäßen Sicherheitsnadelvorrichtung 1 begrenzt. In dieser zweiten Position ist das Ende der Nadel 3 innerhalb der Hülse 9 der Abdeckung 6 angeordnet und tritt nicht durch die Durchbrechung 7 in der Abdeckung 6 hindurch. Die als Feder 12 ausgebildete Verschlusseinrichtung verschließt in dieser zweiten Position die Durchbrechung 7 mittels eines Schenkels 13, da sich die Feder nach dem Entfernen der Nadel 3 aus der Durchbrechung 7 bis zum Anschlag an eine Wandung 11 der Abdeckung 6 entspannt hat, wobei das Ende des Schenkels 13 derart ausgebildet ist, dass es die Durchbrechung 7 in der Abdeckung 6 für die Nadel 3 verschließt.

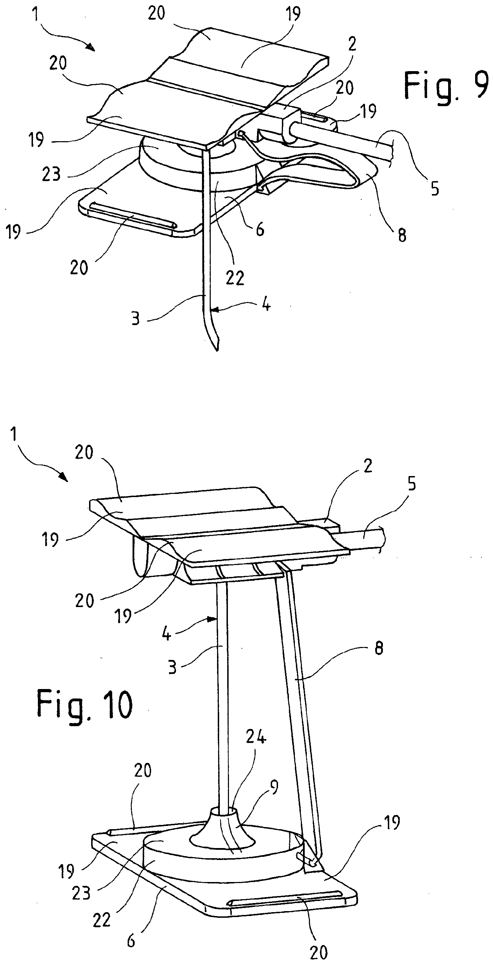

Figur 9 zeigt eine perspektivische Ansicht einer vierten Ausführungsform einer erfindungsgemäßen Sicherheitsnadelvorrichtung 1 in der ersten Position und Figur 10 zeigt eine perspektivische Ansicht der erfindungsgemäßen Sicherheitsnadelvorrichtung 1 aus Figur 9 in der zweiten Position. Die vierte Ausführungsform gemäß der Figuren 9 und 10 unterscheidet sich von der ersten Ausführungsform gemäß der Figuren 1 und 2 dadurch, dass das Begrenzungselement 8 als flexibles Band ausgebildet ist, welches beispielsweise aus Kunststoff besteht und einen rechteckigen Querschnitt aufweist. Alternativ kann das Begrenzungselement 8 auch aus einem geflochtenen oder gewebten Material bestehen, beispielsweise unter Verwendung von einem Textil, Kunststoff oder einem Metall.

Das Begrenzungselement 8 ist an der Abdeckung 6 und an dem ersten Gehäuse 2 fixiert und begrenzt in der zweiten Position, welche in Figur 10 dargestellt ist, die Relativbewegung zwischen dem ersten Gehäuse 2 und der Abdeckung 6 derart, dass die Spitze 3 der Nadel 3 innerhalb der Führungshülse 9 der Abdeckung 6 angeordnet ist.

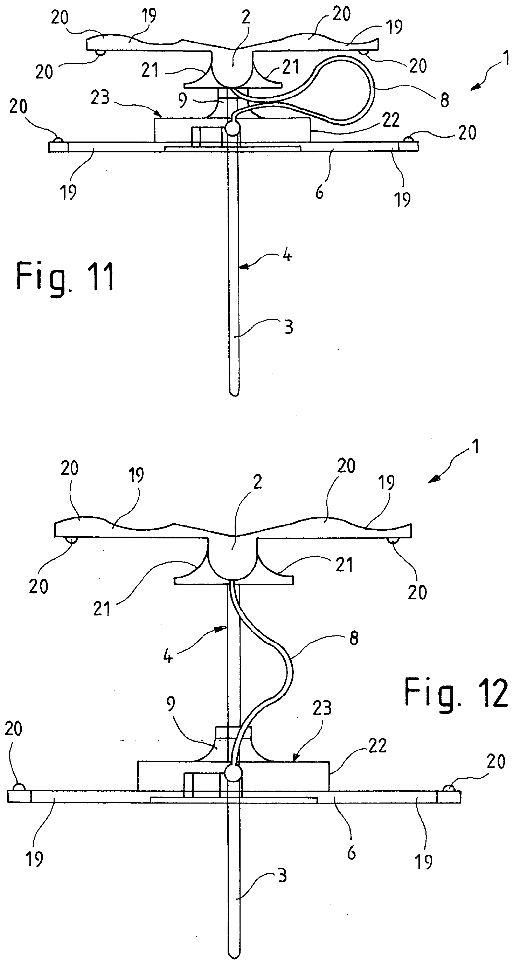

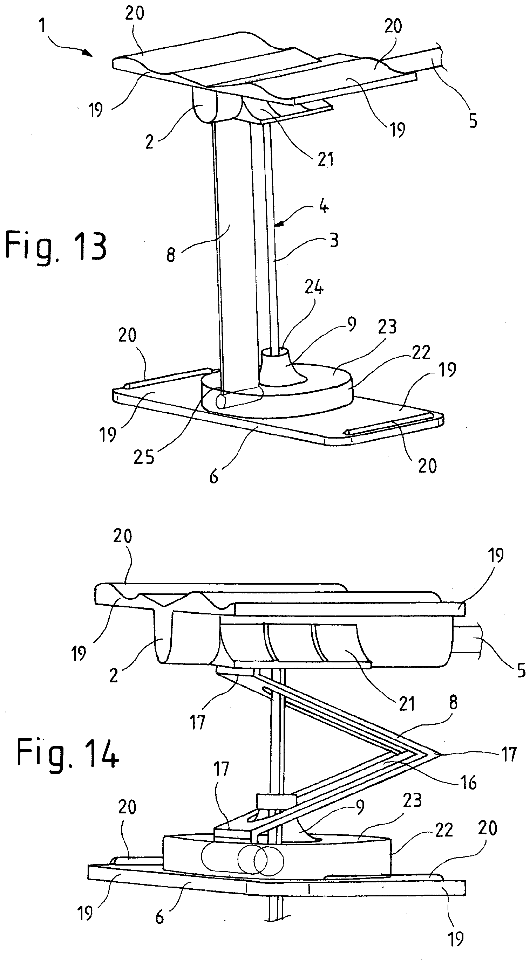

In Figur 11 ist eine Seitenansicht einer fünften Ausführungsform einer erfindungsgemäßen Sicherheitsnadelvorrichtung 1 in der ersten Position dargestellt, in Figur 12 ist eine Seitenansicht der erfindungsgemäßen Sicherheitsnadelvorrichtung 1 aus Figur 11 in einer Zwischenposition und in Figur 13 eine perspektivische Ansicht der erfindungsgemäßen Sicherheitsnadelvorrichtung 1 aus Figur 11 in der zweiten Position dargestellt. Die fünfte Ausführungsform gemäß der Figuren 11 bis 13 unterscheidet sich von der vierten Ausführungsform gemäß der Figuren 9 und 10 durch die Führung des flexiblen Begrenzungselements 8. In der vierten Ausführungsform gemäß der Figuren 9 und 10 legt sich das flexible Begrenzungselement 8 in der ersten Position in Richtung des zweiten Teilabschnitte 5 der Nadel 3 beziehungsweise der Zuführleitung zu dem ersten Gehäuse 2 zusammen, während sich das flexible Begrenzungselement 8 gemäß der fünften Ausführungsform nach den Figuren 11 bis 13 unterhalb der Flügel 19 des ersten Gehäuses 2 der erfindungsgemäßen Sicherheitsnadelvorrichtung 1 zusammenlegt. Weiterhin ist in der vierten Ausführungsform gemäß der Figuren 9 und 10 das Begrenzungselement 8 am Rand der Abdeckung 6 außerhalb des zweiten Gehäuses fixiert, während in der fünften Ausführungsform gemäß der Figuren 11 bis 13 das Begrenzungselement 8 innerhalb des Gehäuses der Abdeckung 6 fixiert ist und durch eine zweite Durchbrechung 25 im Deckel 23 des zweiten Gehäuses austritt.

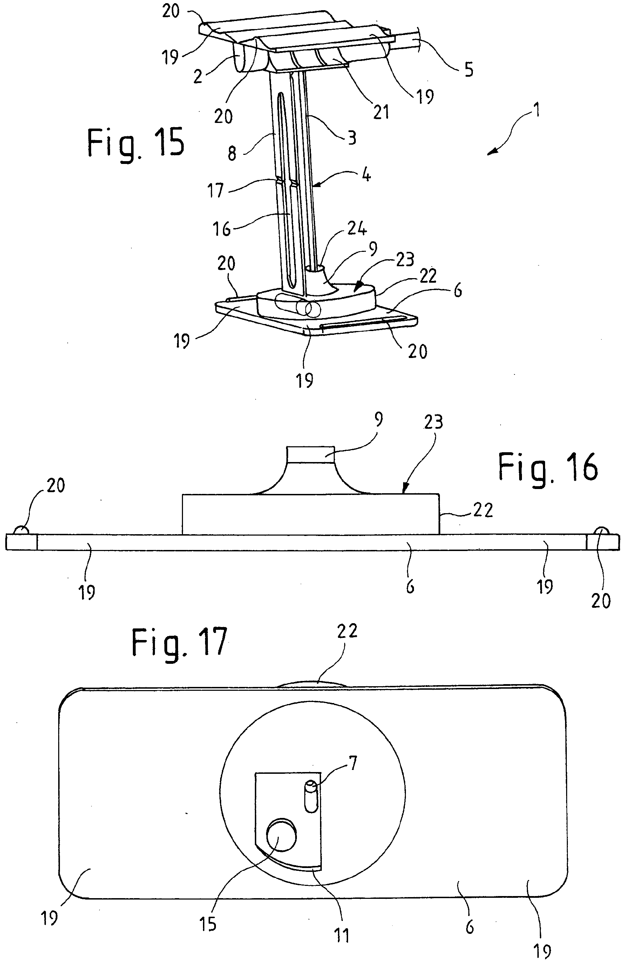

In Figur 14 ist eine perspektivische Ansicht einer sechsten Ausführungsform einer erfindungsgemäßen Sicherheitsnadelvorrichtung 1 in der Zwischenposition dargestellt. In Figur 15 ist eine perspektivische Ansicht der erfindungsgemäßen Sicherheitsnadelvorrichtung 1 aus Figur 14 in der zweiten Position dargestellt. Die sechste Ausführungsform gemäß der Figuren 14 und 15 unterscheidet sich von der vierten Ausführungsform der erfindungsgemäßen Sicherheitsnadelvorrichtung 1 aus den Figuren 9 und 10 dadurch, dass das Begrenzungselement 8 zwei starre Abschnitte aufweist, welche zwischen insgesamt drei Filmgelenken sitzend angeordnet sind. Weiterhin umfasst das Begrenzungselement 8 einen geschlitzten Bereich 16, in welchem der erste Teilabschnitt 4 der Nadel 3 wenigstens in der ersten Position angeordnet ist, vorzugsweise ebenfalls während der Relativbewegung zwischen dem ersten Gehäuse 2 und der Abdeckung 6 bis kurz vor dem Erreichen der zweiten Position. In der ersten Position ist das Begrenzungselement 8 im Wesentlichen parallel zu der Oberfläche des menschlichen oder tierischen Körpers angeordnet. Weiterhin ist das Begrenzungselement 8 wie in der fünften Ausführungsform gemäß der Figuren 11 und 12 innerhalb des zweiten Gehäuses der Abdeckung 6 angeordnet.

Figur 16 zeigt eine seitliche Detailansicht einer Abdeckung 6 von einer erfindungsgemäßen Sicherheitsnadelvorrichtung 1. In Figur 17 ist eine Detailansicht von der dem menschlichen oder tierischen Körper zugewandten Seite auf die Abdeckung 6 aus Figur 16 dargestellt. Der Figur 17 ist insbesondere zu entnehmen, dass die Abdeckung 6 einen Hohlraum mit Seitenwänden 11 aufweist, in welchem die Verschlusseinrichtung 10 angeordnet werden kann. Weiterhin ist innerhalb des Hohlraums eine als zylindrische Erhebung ausgebildete Fixierung 15 für die Verschlusseinrichtung angeordnet.

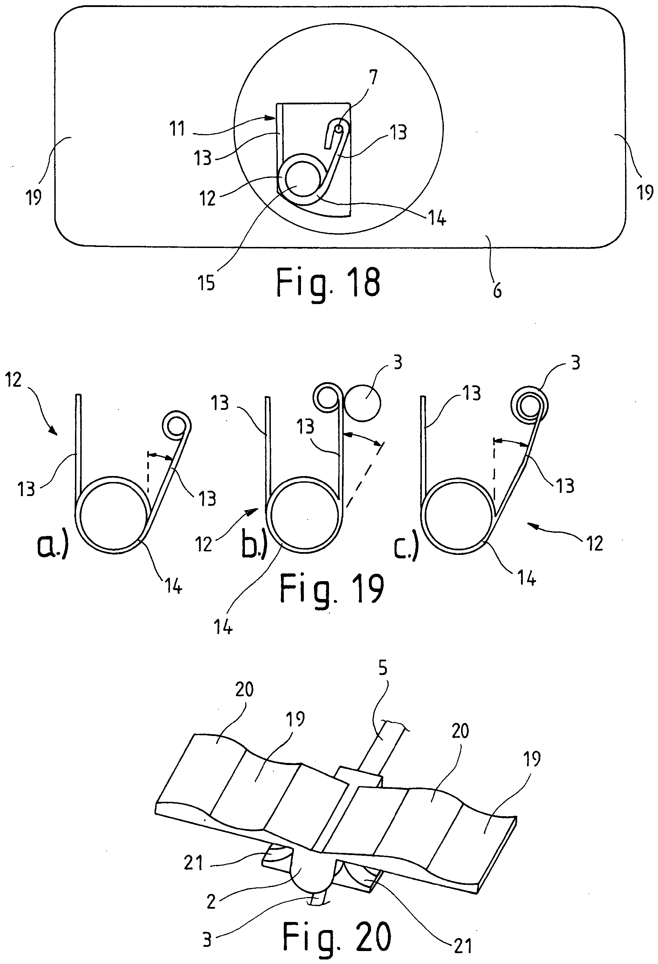

Figur 18 zeigt eine Detailansicht gemäß Figur 17 mit einer in der Abdeckung 6 angeordneten Verschlusseinrichtung 10. Die Verschlusseinrichtung 10 ist als Feder 12 ausgebildet und umfasst zwei Schenkel 13 und einen Wicklungsbereich 14, wobei die zylindrische Erhebung 15 in die Wicklung 14 der Feder 12 eingreift. In Figur 15 ist die Abdeckung 6 in der zweiten Position dargestellt, in welcher der eine Schenkel 13 der Feder 12 die Durchbrechung 7 in der Abdeckung 6 für die Nadel 3 verschließt.

Figur 19 zeigt Detailansichten einer als Feder 12 ausgebildeten Verschlusseinrichtung 10 zur Verwendung mit einer erfindungsgemäßen Sicherheitsnadelvorrichtung 1. In Figur 19 a ist die Feder 12 in einem entspannten Zustand dargestellt. Die Schenkel 13 der Nadel 12 weisen dabei eine Länge von 6 mm auf. Im Bereich der Durchbrechung 7 der Abdeckung 6 weist der eine Schenkel 13 eine weitere Aufwicklung auf, um die Durchbrechung 7 für die Nadel 3 zu verschließen. Der Wicklungsbereich 14 der Feder 12 weist beispielsweise 2,42 Wicklungen auf und hat einen inneren Durchmesser von 4 mm.

In Figur 19 b ist die Feder 12 in der ersten Position der Sicherheitsnadelvorrichtung 1 dargestellt. In dieser ersten Position liegt der eine Schenkel 13 an einer Seitenwandung der Abdeckung 6 an und der andere Schenkel 13 der Feder 12 liegt an dem Schaft des ersten Teilabschnitts 4 der Nadel 3 an, so dass die Feder 12 in einem gespannten Zustand ist. In diesem gespannten Zustand entsteht eine Reibungskraft zwischen dem Schenkel 13 und dem Schaft der Nadel 3, wodurch beispielsweise eine Relativbewegung zwischen dem ersten Gehäuse 2 und der Abdeckung 6 der erfindungsgemäßen Sicherheitsnadelvorrichtung 1 verhindert wird.

Figur 19c zeigt die Feder 12 in der zweiten Position der erfindungsgemäßen Sicherheitsnadelvorrichtung 1. In dieser zweiten Position liegen beide Schenkel 13 der Feder 12 an einer Seitenwandung der Abdeckung 6 an, wobei die Feder 12 in einem leicht gespannten Zustand ist und der eine Schenkel 13 die Durchbrechung 7 in der Abdeckung 6 für die Nadel 3 verschließt.

Figur 20 zeigt eine Detailansicht eines ersten Gehäuses 2 von einer erfindungsgemäßen Sicherheitsnadelvorrichtung 1. Der Figur 20 lassen sich insbesondere die Ausbuchtungen 21 für die Finger eines Benutzers entnehmen, die unterhalb der Flügel 19 angeordnet sind. Weiterhin lässt sich der Figur 20 die komplementär zueinander ausgebildeten Oberflächen der Flügel 19 entnehmen. Die komplementär zueinander ausgebildeten Oberflächen der Flügel 19 sind in der ersten Position auf der dem menschlichen oder tierischen Körper abgewandten Oberfläche der Flügel 19 angeordnet und greifen bei einem Zusammenklappen der Flügel 19 ineinander.

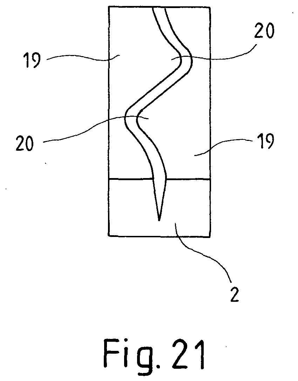

Figur 21 zeigt eine Schnittansicht der gefalteten Flügel 19 eines ersten Gehäuses 2 von einer erfindungsgemäßen Sicherheitsnadelvorrichtung 1. In dieser Position greifen die komplementär zueinander ausgebildeten Oberflächen der Flügel 19 ineinander, so dass das Bedienpersonal das Gefühl vermittelt bekommt, dass es sich dabei um ein einziges Element handelt, was die Benutzung der erfindungsgemäßen Sicherheitsnadelvorrichtung 1 verbessert.

### Bezugszeichenliste

- 1: Sicherheitsnadelvorrichtung
- 2: erstes Gehäuse
- 3: hohle Nadel
- 4: erster Teilabschnitt
- 5: zweiter Teilabschnitt
- 6: Abdeckung
- 7: Durchbrechung
- 8: Begrenzungselement
- 9: Führungshülse
- 10: Verschlusseinrichtung
- 11: Wandung der Abdeckung
- 12: Feder
- 13: Schenkel
- 14: Wicklungsbereich
- 15: Fixierung
- 16: geschlitzter Bereich des Begrenzungselements
- 17: Filmgelenk
- 18: Hohlraum der Abdeckung
- 19: Flügel
- 20: Rippe
- 21: Ausbuchtung für Finger
- 22: Seitenwände
- 23: Deckel
- 24: erste Durchbrechung im Deckel
- 25: zweite Durchbrechung im Deckel

## Patentansprüche

1. Sicherheitsnadelvorrichtung (1), insbesondere zur Punktion von in einem menschlichen oder tierischen Körper subkutan implantierten Port, umfassend:
ein erstes Gehäuse (2);
eine hohle Nadel (3), wobei die Nadel (3) einen ersten Teilabschnitt (4) und einen zweiten Teilabschnitt (5) aufweist und sich der erste Teilabschnitt (4) entlang einer Punktionsachse erstreckt, die rechtwinklig zu der Oberfläche des menschlichen oder tierischen Körpers verläuft, und der zweite Teilabschnitt (5) der Nadel (3) rechtwinklig zu dem ersten Teilabschnitt (4) angeordnet ist, im Wesentlichen parallel zu der Oberfläche des menschlichen oder tierischen Körpers, wobei die hohle Nadel (3) mit dem ersten Gehäuse (2) verbunden oder verbindbar ist, insbesondere im Bereich des zweiten Teilabschnitts (5);
eine Abdeckung (6) mit einer Durchbrechung (7) zur Aufnahme des ersten Teilabschnitts (4) der Nadel (3), wobei die Abdeckung (6) von einer ersten Position in eine zweite Position relativ zu dem ersten Gehäuse (2) bewegbar ist, wobei die Bewegung im Wesentlichen entlang der Punktionsachse erfolgt; und
ein wenigstens teilweise flexibles Begrenzungselement (8), zum Begrenzen der Relativbewegung zwischen dem ersten Gehäuse (2) und der Abdeckung (6) in der zweiten Position,
**dadurch gekennzeichnet, dass**
die Abdeckung (6) auf der dem menschlichen oder tierischen Körper abgewandten Oberfläche eine Führungshülse (9) für den ersten Teilabschnitt (4) der Nadel (3) aufweist, zur Führung der Relativbewegung zwischen der Abdeckung (6) und dem ersten Gehäuse (2), so dass die Nadel (3) in Richtung der Punktionsachse aus dem Port entfernt wird, wobei die Nadelspitze in der zweiten Position innerhalb der Führungshülse (9) angeordnet ist,
und **dadurch gekennzeichnet, dass**
das erste Gehäuse (2) zwei Flügel (19) umfasst, welche aus einer ersten Ausrichtung, welche im Wesentlichen rechtwinklig zur Punktionsachse verläuft, in eine zweite Ausrichtung, im Wesentlichen parallel zur Punktionsachse, klappbar sind, wobei die in der ersten Ausrichtung dem menschlichen oder tierischen Körper abgewandten Oberflächen der Flügel (19) in der zweiten Ausrichtung direkt benachbart zueinander angeordnet und komplementär zueinander ausgebildet sind.

2. Sicherheitsnadelvorrichtung (1) nach Anspruch 1, **dadurch gekennzeichnet, dass** das erste Gehäuse (2) und die Abdeckung (6) in der ersten Position direkt benachbart zueinander angeordnet sind, wobei die Spitze der Nadel (3) in der ersten Position aus der dem menschlichen oder tierischen Körper zugewandten Oberfläche der Abdeckung (6) austritt.

3. Sicherheitsnadelvorrichtung (1) nach Anspruch 1 oder 2, **dadurch gekennzeichnet, dass** das erste Gehäuse (2) und die Abdeckung (6) in der ersten Position und/oder der zweiten Position relativ zueinander fixierbar sind.

4. Sicherheitsnadelvorrichtung (1) nach einem der Ansprüche 1 bis 3, **dadurch gekennzeichnet, dass** die Sicherheitsnadelvorrichtung (1) weiterhin eine Verschlusseinrichtung (10) für die Durchbrechung (7) in der Abdeckung (6) umfasst, welche die Durchbrechung (7) für die Nadel (3) in der zweiten Position verschließt, wobei die Verschlusseinrichtung (10) vorzugsweise als Feder (12) ausgebildet ist, welche in der ersten Position zwischen einer Oberfläche der Abdeckung (6), vorzugsweise einer Wandung (11), und der Nadel (3) angeordnet ist und wobei sich die Feder (12) bei Erreichen der zweiten Position, in welcher die Nadel (3) nicht aus der Durchbrechung (7) in der Abdeckung (6) austritt, entspannt und die Durchbrechung (7) für die Nadel (3) verschließt.

5. Sicherheitsnadelvorrichtung (1) nach Anspruch 4, **dadurch gekennzeichnet, dass** das Abdeckelement (6) eine Fixierung (15) für die Verschlusseinrichtung (10) aufweist, zur Fixierung der Verschlusseinrichtung (10) am Abdeckelement (6), beispielsweise in Form einer zylindrischen Erhebung, die in die Wicklung (14) der Feder (12) eingreift.

6. Sicherheitsnadelvorrichtung (1) nach einem der Ansprüche 1 bis 5, **dadurch gekennzeichnet, dass** das flexible Begrenzungselement (8) derart ausgebildet ist, dass die Spitze der Nadel (3) in der zweiten Position innerhalbes der Führungshülse (9) angeordnet ist.

7. Sicherheitsnadelvorrichtung (1) nach Anspruch 8, **dadurch gekennzeichnet, dass** das Begrenzungselement (8) schlauchförmig, insbesondere konisch, ausgebildet ist und der erste Teilabschnitt (4) der Nadel (3) wenigstens teilweise innerhalb des schlauchförmigen Begrenzungselements (8) angeordnet ist oder dass das Begrenzungselement (8) aus wenigstens einem fadenförmigen oder bandförmigen Element ausgebildet ist.

8. Sicherheitsnadelvorrichtung (1) nach einem der Ansprüche 7, **dadurch gekennzeichnet, dass** das Begrenzungselement (8) als geschlitztes Band ausgebildet ist, wobei die Nadel (3) wenigstens in der ersten Position in den geschlitzten Bereich (16) des randförmigen Begrenzungselements (8) angeordnet ist, wobei das Begrenzungselement (8) vorzugsweise Filmgelenke (17) aufweist, welche derart angeordnet sind, dass sich das Begrenzungselement (8) in der ersten Position im Wesentlichen parallel zu der Oberfläche des menschlichen oder tierischen Körpers zusammenlegen lässt.

9. Sicherheitsnadelvorrichtung (1) nach einem der Ansprüche 1 bis 8, **dadurch gekennzeichnet, dass** das Begrenzungselement (8) an dem ersten Gehäuse (2) und/oder der Abdeckung (6) befestigt ist, beispielsweise durch eine Klebverbindung oder eine Klemmverbindung.

10. Sicherheitsnadelvorrichtung (1) nach einem der Ansprüche 1 bis 9, **dadurch gekennzeichnet, dass** die Abdeckung (6) wenigstens einen Flügel (19) vorzugsweise zwei Flügel (19), umfasst, wobei der Flügel (19) vorzugsweise wenigstens eine Rippe (20) aufweist.

11. Sicherheitsnadelvorrichtung (1) nach Anspruch 10, **dadurch gekennzeichnet, dass** an dem ersten Gehäuse (2) und an der Abdeckung (6) jeweils zwei Flügel (19) angeordnet sind, wobei die Flügel (19) an dem ersten Gehäuse (2) vorzugsweise größer oder kleiner ausgebildet sind als die Flügel (19) an der Abdeckung (6).

12. Sicherheitsnadelvorrichtung (1) nach Anspruch 10 oder Anspruch 11, **dadurch gekennzeichnet, dass** die Flügel (19) aus einer ersten Ausrichtung, welche im Wesentlichen rechtwinklig zur Punktionsachse verläuft, in eine zweite Ausrichtung, im Wesentlichen parallel zur Punktionsachse, klappbar sind.

13. Sicherheitsnadelvorrichtung (1) nach einem der Ansprüche 1 bis 12, **dadurch gekennzeichnet, dass** das erste Gehäuse (2) wenigstens eine Ausbuchtung (21), vorzugsweise zwei Ausbuchtungen (21), aufweist, zur Aufnahme von Fingern des Bedienpersonals während der Punktion des implantierten Ports, wobei die Ausbuchtung (21) vorzugsweise im Bereich der Punktionsachse angeordnet ist.

14. Sicherheitsnadelvorrichtung (1) nach einem der Ansprüche 1 bis 13, **dadurch gekennzeichnet, dass** die Abdeckung (6) Seitenwände (22) und einen Deckel (23) umfasst, welche zusammen mit der Abdeckung (6) ein zweites Gehäuse ausbilden, wobei der Deckel (23) eine erste Durchbrechung (24) für die Nadel (3) und/oder eine zweite Durchbrechung (25) für das flexible Begrenzungselement (8) aufweist.

15. Sicherheitsnadelvorrichtung (1) nach einem der Ansprüche 1 bis 14, **dadurch gekennzeichnet, dass** die Abdeckung (6) und/oder das erste Gehäuse (2) wenigstens teilweise transparent ausgebildet ist.

## Claims

1. A safety needle device (1), particularly for puncturing a port implanted subcutaneously in a human or animal body, comprising:
a first housing (2);
a hollow needle (3), wherein the needle (3) comprising a first sub-section (4) and a second sub-section (5), the first sub-section (4) extends along a puncture axis, which runs at a right angle to the surface of the human or animal body, and the second sub-section (5) of the needle (3) being arranged at a right angle to the first sub-section (4), substantially in parallel to the surface of the human or animal body, wherein the hollow needle (3) being connected or connectable to the first housing (2), particularly in the region of the second sub-section (5);
a cover (6) comprising a through-hole (7) for receiving said first sub-section (4) of the needle (3), wherein the cover (6) is being movable from a first position to a second position relative to said first housing (2), wherein the movement being substantially along said puncture axis; and
an at least partially flexible limiting element (8) which limiting the relative movement between the first housing (2) and the cover (6) in the second position,
**characterised in that**
the cover (6) comprises on the surface facing away from the human or animal body a guide sleeve (9) for the first sub-section (4) of the needle (3), for guiding the relative movement between the cover (6) and the first housing (2), so that the needle (3) is removed from the port in the direction of the puncture axis, wherein the needle tip being arranged inside the guide sleeve (9) in the second position,
and **characterised in that**
the first housing (2) comprises two wings (19), which are foldable from a first orientation, which extends substantially perpendicular to the puncture axis, into a second orientation, substantially parallel to the puncture axis, wherein the surfaces of the wings (19) facing away from the human or animal body in the first orientation are arranged directly adjacent to one another in the second orientation and are complementary to one another.

2. Safety needle device (1) according to claim 1, **characterized in that** the first housing (2) and the cover (6) are arranged directly adjacent to one another in the first position, wherein the tip of the needle (3) in the first position emerges from the surface of the cover (6) facing the human or animal body.

3. Safety needle device (1) according to claim 1 or 2, **characterized in that** the first housing (2) and the cover (6) are fixable relative to each another in the first position and/or the second position.

4. Safety needle device (1) according to one of claims 1 to 3, **characterized in that** the safety needle device (1) further comprises a closure means (10) for the through-hole (7) in the cover (6), which closes the through-hole (7) for the needle (3) in the second position, wherein the closure means (10) preferably is designed as a spring (12), which is arranged in the first position between a surface of the cover (6), preferably a wall (11), and the needle (3), and wherein the spring (12) is unstressed on reaching the second position, in which the needle (3) does not emerge from the through-hole (7) in the cover (6), and closes the through-hole (7) for the needle (3).

5. Safety needle device (1) according to claim 4, **characterized in that** the cover element (6) has a fixing (15) for the closure means (10) for fixing the closure means (10) to the cover element (6), for example in the form of a cylindrical elevation which engages in the winding (14) of the spring (12).

6. Safety needle device (1) according to one of claims 1 to 5, **characterized in that** the flexible limiting element (8) is designed such that the tip of the needle (3) is arranged in the second position inside the guide sleeve (9).

7. Safety needle device (1) according to claim 8, **characterised in that** the limiting element (8) is tubular, in particular conical, and the first sub-section (4) of the needle (3) is at least partially arranged inside the tubular limiting element (8) or that the limiting element (8) is formed from at least one thread-like or band-shaped element.

8. Safety needle device (1) according to one of the claims 7, **characterized in that** the limiting element (8) is formed as a slotted strip, wherein the needle (3) is arranged at least in the first position in the slotted portion (16) of the band-shaped limiting element (8), wherein the limiting element (8) preferably comprises film hinges (17) which are arranged in such a way that the limiting element (8) is foldable in the first position substantially in parallel to the surface of the human or animal body.

9. Safety needle device (1) according to one of claims 1 to 8, **characterized in that** the limiting element (8) is attached to the first housing (2) and/or the cover (6), for example by an adhesive connection or a clamping connection.

10. Safety needle device (1) according to one of claims 1 to 9, **characterized in that** the cover (6) comprises at least one wing (19), preferably two wings (19), wherein the wing (19) preferably has at least one rib (20).

11. Safety needle device (1) according to claim 10, **characterized in that** two respective wings (19) are arranged on the first housing (2) and on the cover (6), wherein the wings (19) on the first housing (2) preferably being larger or smaller than the wings (19) on the cover (6).

12. Safety needle device (1) according to claim 10 or claim 11, **characterized in that** the wings (19) are foldable from a first orientation, which extends substantially perpendicular to the puncture axis, into a second orientation, substantially parallel to the puncture axis.

13. Safety needle device (1) according to one of the claims 1 to 12, **characterized in that** the first housing (2) has at least one convexity (21), preferably two convexities (21), for receiving the fingers of the operating personnel during the puncture of the implanted port, wherein the convexity (21) is preferably arranged in the region of the puncture axis.

14. Safety needle device (1) according to one of claims 1 to 13, **characterized in that** the cover (6) comprises side walls (22) and a cap (23), which together with the cover (6) form a second housing, wherein the cap (23) has a first opening (24) for the needle (3) and/or a second opening (25) for the flexible limiting element (8).

15. Safety needle device (1) according to one of claims 1 to 14, **characterized in that** the cover (6) and/or the first housing (2) is at least partially transparent.

## Revendications

1. Dispositif d'aiguille de sécurité (1), destiné en particulier à percer un orifice implanté de manière sous-cutanée dans un corps humain ou animal, comprenant :
un premier boîtier (2) ;
une aiguille creuse (3), dans lequel l'aiguille (3) comprend une première sous-section (4) et une seconde sous-section (5), la première sous-section (4) s'étend le long d'un axe de ponction, qui s'étend selon un angle droit à la surface du corps humain ou animal, et la seconde sous-section (5) de l'aiguille (3) étant agencée selon un angle droit à la première sous-section (4), sensiblement en parallèle à la surface du corps humain ou animal, dans lequel l'aiguille creuse (3) est connectée ou peut être connectée au premier boîtier (2), particulièrement dans la région de la seconde sous-section (5) ;
un couvercle (6) comprenant un trou traversant (7) permettant de recevoir ladite première sous-section (4) de l'aiguille (3), dans lequel le couvercle (6) peut être déplacé d'une première position à une seconde position par rapport audit premier boîtier (2), dans lequel le mouvement s'effectue sensiblement le long dudit axe de ponction ; et
un élément de limitation au moins partiellement flexible (8) qui limite le mouvement relatif entre le premier boîtier (2) et le couvercle (6) dans la seconde position,
**caractérisé en ce que**
le couvercle (6) comprend, sur la surface orientée à l'opposé du corps humain ou animal, un manchon de guidage (9) pour la première sous-section (4) de l'aiguille (3), permettant de guider le mouvement relatif entre le couvercle (6) et le premier boîtier (2), de sorte que l'aiguille (3) est retirée de l'orifice dans la direction de l'axe de ponction, dans lequel la pointe de l'aiguille est agencée à l'intérieur du manchon de guidage (9) dans la seconde position,
et **caractérisé en ce que**
le premier boîtier (2) comprend deux ailes (19), qui sont pliables à partir d'une première orientation, qui s'étend sensiblement perpendiculaire à l'axe de ponction, dans une seconde orientation, sensiblement parallèle à l'axe de ponction, dans lequel les surfaces des ailes (19) orientées à l'opposé du corps humain ou animal dans la première orientation sont agencées directement adjacentes les unes aux autres dans la seconde orientation et sont complémentaires les unes des autres.

2. Dispositif d'aiguille de sécurité (1) selon la revendication 1, **caractérisé en ce que** le premier boîtier (2) et le couvercle (6) sont agencés directement adjacents l'un à l'autre dans la première position, dans lequel la pointe de l'aiguille (3) dans la première position émerge à partir de la surface du couvercle (6) orientée vers le corps humain ou animal.

3. Dispositif d'aiguille de sécurité (1) selon la revendication 1 ou 2, **caractérisé en ce que** le premier boîtier (2) et le couvercle (6) peuvent être fixés l'un par rapport à l'autre dans la première position et/ou la seconde position.

4. Dispositif d'aiguille de sécurité (1) selon l'une des revendications 1 à 3, **caractérisé en ce que** le dispositif d'aiguille de sécurité (1) comprend en outre un moyen de fermeture (10) pour le trou traversant (7) dans le couvercle (6), qui ferme le trou traversant (7) pour l'aiguille (3) dans la seconde position, dans lequel le moyen de fermeture (10) est conçu de préférence comme un ressort (12), qui est agencé dans la première position entre une surface du couvercle (6), de préférence une paroi (11), et l'aiguille (3), et dans lequel le ressort (12) est sans contrainte lorsqu'il atteint la seconde position, dans laquelle l'aiguille (3) n'émerge pas du trou traversant (7) dans le couvercle (6), et ferme le trou traversant (7) pour l'aiguille (3).

5. Dispositif d'aiguille de sécurité (1) selon la revendication 4, **caractérisé en ce que** l'élément de couvercle (6) possède une fixation (15) pour le moyen de fermeture (10) permettant de fixer le moyen de fermeture (10) à l'élément de couvercle (6), par exemple sous la forme d'une élévation cylindrique qui entre en prise dans l'enroulement (14) du ressort (12).

6. Dispositif d'aiguille de sécurité (1) selon l'une des revendications 1 à 5, **caractérisé en ce que** l'élément de limitation flexible (8) est conçu de telle sorte que la pointe de l'aiguille (3) soit agencée dans la seconde position à l'intérieur du manchon de guidage (9).

7. Dispositif d'aiguille de sécurité (1) selon la revendication 8, **caractérisé en ce que** l'élément de limitation (8) est tubulaire, en particulier conique, et la première sous-section (4) de l'aiguille (3) est au moins partiellement agencée à l'intérieur de l'élément de limitation tubulaire (8) ou **en ce que** l'élément de limitation (8) est formé à partir d'au moins un élément en forme de fil ou de bande.

8. Dispositif d'aiguille de sécurité (1) selon la revendication 7, **caractérisé en ce que** l'élément de limitation (8) est sous la forme d'une bande fendue, dans lequel l'aiguille (3) est agencée au moins dans la première position dans la partie fendue (16) de l'élément de limitation en forme de bande (8), dans lequel l'élément de limitation (8) comprend de préférence des charnières de films (17) qui sont agencées de telle sorte que l'élément de limitation (8) peut être plié dans la première position sensiblement en parallèle à la surface du corps humain ou animal.

9. Dispositif d'aiguille de sécurité (1) selon l'une des revendications 1 à 8, **caractérisé en ce que** l'élément de limitation (8) est fixé au premier boîtier (2) et/ ou au couvercle (6), par exemple par une liaison adhésive ou une liaison de serrage.

10. Dispositif d'aiguille de sécurité (1) selon l'une des revendications 1 à 9, **caractérisé en ce que** le couvercle (6) comprend au moins une aile (19), de préférence deux ailes (19), dans lequel l'aile (19) possède de préférence au moins une nervure (20).

11. Dispositif d'aiguille de sécurité (1) selon la revendication 10, **caractérisé en ce que** deux ailes respectives (19) sont agencées sur le premier boîtier (2) et sur le couvercle (6), dans lequel les ailes (19) sur le premier boîtier (2) sont de préférence plus grandes ou plus petites que les ailes (19) sur le couvercle (6).

12. Dispositif d'aiguille de sécurité (1) selon la revendication 10 ou la revendication 11, **caractérisé en ce que** les ailes (19) sont pliables à partir d'une première orientation, qui s'étend sensiblement perpendiculaire à l'axe de ponction, dans une seconde orientation, sensiblement parallèle à l'axe de ponction.

13. Dispositif d'aiguille de sécurité (1) selon l'une des revendications 1 à 12, **caractérisé en ce que** le premier boîtier (2) possède au moins une convexité (21), de préférence deux convexités (21), permettant de recevoir les doigts du personnel de service pendant la ponction de l'orifice implanté, dans lequel la convexité (21) est agencée de préférence dans la région de l'axe de ponction.

14. Dispositif d'aiguille de sécurité (1) selon l'une des revendications 1 à 13, **caractérisé en ce que** le couvercle (6) comprend des parois latérales (22) et un capuchon (23), qui, conjointement au couvercle (6), forment un second boîtier, dans lequel le capuchon (23) possède une première ouverture (24) pour l'aiguille (3) et/ou une seconde ouverture (25) pour l'élément de limitation flexible (8).

15. Dispositif d'aiguille de sécurité (1) selon l'une des revendications 1 à 14, **caractérisé en ce que** le couvercle (6) et/ou le premier boîtier (2) est au moins partiellement transparent.
